(19)

Europäisches
Patentamt
European
Patent Office
Office européen
des brevets

(11) **EP 3 950 071 A1**

(12) **EUROPEAN PATENT APPLICATION**
published in accordance with Art. 153(4) EPC

(43) Date of publication:
**09.02.2022 Bulletin 2022/06**

(21) Application number: 20782831.0

(22) Date of filing: **27.03.2020**

(51) International Patent Classification (IPC):
$A61Q\ 19/00^{(2006.01)}$ $\quad A61K\ 9/06^{(2006.01)}$
$A61K\ 9/50^{(2006.01)}$ $\quad A61K\ 9/70^{(2006.01)}$
$A61L\ 15/20^{(2006.01)}$ $\quad A61L\ 15/44^{(2006.01)}$
$A61L\ 26/00^{(2006.01)}$ $\quad A61K\ 8/11^{(2006.01)}$
$A61K\ 8/37^{(2006.01)}$ $\quad A61K\ 8/42^{(2006.01)}$
$A61K\ 47/14^{(2017.01)}$ $\quad A61K\ 47/18^{(2017.01)}$
$A61K\ 47/26^{(2006.01)}$ $\quad A61K\ 31/53^{(2006.01)}$

(52) Cooperative Patent Classification (CPC):
A61K 8/11; A61K 8/37; A61K 8/42; A61K 9/06;
A61K 9/50; A61K 9/70; A61K 31/53; A61K 47/14;
A61K 47/18; A61K 47/26; A61L 15/20;
A61L 15/44; A61L 26/00; A61Q 19/00

(86) International application number:
**PCT/JP2020/013934**

(87) International publication number:
**WO 2020/203733 (08.10.2020 Gazette 2020/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.03.2019 JP 2019067517**

(71) Applicant: **SEKISUI CHEMICAL CO., LTD.
Osaka-shi
Osaka
530-8565 (JP)**

(72) Inventors:
• **KAWAMURA, Daichi
Mishima-gun, Osaka 618-0021 (JP)**

• **MATSUMOTO, Izumi
Mishima-gun, Osaka 618-0021 (JP)**
• **AKAMINE, Takayuki
Mishima-gun, Osaka 618-0021 (JP)**
• **TONE, Saori
Mishima-gun, Osaka 618-0021 (JP)**
• **NAKAMURA, Yuuta
Mishima-gun, Osaka 618-0021 (JP)**
• **LI, Yan
Mishima-gun, Osaka 618-0021 (JP)**
• **OKAMOTO, Naoki
Mishima-gun, Osaka 618-0021 (JP)**

(74) Representative: **Ter Meer Steinmeister & Partner
Patentanwälte mbB
Nymphenburger Straße 4
80335 München (DE)**

(54) **CORE-SHELL STRUCTURE, PREPARATION, MEDICINE FOR EXTERNAL APPLICATION, TAPE AGENT AND COSMETIC PRODUCT**

(57) Provided is a core-shell structure that can achieve both improvement in transdermal absorption of an active ingredient and reduction in skin irritation at a high level. A core-shell structure 10 includes: a core portion 11 containing an active ingredient, the core portion being solid; and a shell portion 12 containing both a first surfactant having a melting point of less than 35°C and a second surfactant having a melting point of 35°C or more, the first surfactant and the second surfactant each having an HLB value of 4 to 14, the first surfactant and the second surfactant each containing at least one selected from the group consisting of sorbitan fatty acid esters, glycerin fatty acid esters, propylene glycol fatty acid esters, and fatty acid alkanolamides.

EP 3 950 071 A1

[FIG. 1.]

10

12

11

**Description**

**TECHNICAL FIELD**

The present invention relates to a core-shell structure, and a preparation, an external medicine, a tape preparation, and a cosmetic that contain the core-shell structure.

**BACKGROUND ART**

In the fields of external medicines, cosmetics, and the like, techniques for transdermal absorption of an active ingredient such as a drug have been developed. It is known that the process for transdermal absorption of an active ingredient is sometimes influenced by skin barrier function, metabolism, or the like, and that the influence depends on drugs.

Patent Document 1 described below discloses a core-shell structure including a core portion containing an active ingredient and including a shell portion containing a surfactant having a hydrophile lipophile balance (HLB) value of 4 to 14. Patent Document 1 describes that the surfactant has a saturated hydrocarbon group having 7 to 15 carbon atoms or an unsaturated hydrocarbon group having 7 to 17 carbon atoms.

**Related Art Document**

**Patent Document**

Patent Document 1: WO 2018/147333 A

**SUMMARY OF THE INVENTION**

**PROBLEMS TO BE SOLVED BY THE INVENTION**

A preparation containing a core-shell structure as disclosed in Patent Document 1 has an excellent immediate effect in transdermal absorption of the active ingredient. However, when the transdermal absorption of the active ingredient is improved in the preparation of Patent Document 1, the skin irritation is increased. As a result, a side effect such as a skin symptom is sometimes caused at the application site of the preparation. Therefore, it is needed to achieve both improvement in transdermal absorption of an active ingredient and reduction in skin irritation at a further high level.

An object of the present invention is to provide a core-shell structure, a preparation, an external medicine, a tape preparation, and a cosmetic that can achieve both improvement in transdermal absorption of an active ingredient and reduction in skin irritation at a high level.

**MEANS FOR SOLVING THE PROBLEMS**

The core-shell structure according to the present invention includes: a core portion containing an active ingredient, the core portion being solid; and a shell portion containing a first surfactant having a melting point of less than 35°C and a second surfactant having a melting point of 35°C or more, the first surfactant and the second surfactant each having an HLB value of 4 to 14, the first surfactant and the second surfactant each containing at least one selected from the group consisting of sorbitan fatty acid esters, glycerin fatty acid esters, propylene glycol fatty acid esters, and fatty acid alkanolamides.

In a specific aspect of the core-shell structure according to the present invention, a mass ratio of the first surfactant to the second surfactant (the first surfactant : the second surfactant) is 10 : 90 to 80 : 20.

In another specific aspect of the core-shell structure according to the present invention, the first surfactant and the second surfactant each have a saturated hydrocarbon group having 5 to 15 carbon atoms or an unsaturated hydrocarbon group having 5 to 17 carbon atoms.

In another specific aspect of the core-shell structure according to the present invention, each hydrocarbon group in the first surfactant has 5 or more and 8 or less carbon atoms.

In still another specific aspect of the core-shell structure according to the present invention, each hydrocarbon group in the second surfactant has 9 or more and 17 or less carbon atoms.

In still another specific aspect of the core-shell structure according to the present invention, the first surfactant and the second surfactant each include an ester bond or an amide bond formed between an alcohol and a fatty acid, and the alcohol has a molecular weight of 70 g/mol or more and 330 g/mol or less.

In still another specific aspect of the core-shell structure according to the present invention, the first surfactant and the second surfactant each contain at least one selected from the group consisting of sorbitan fatty acid esters, glycerin fatty acid esters, and propylene glycol fatty acid esters.

In still another specific aspect of the core-shell structure according to the present invention, a glycerin fatty acid ester contained in each of the first surfactant and the second surfactant is at least one selected from the group consisting of monoglycerin fatty acid esters, diglycerin fatty acid esters, and triglycerin fatty acid esters.

In still another specific aspect of the core-shell structure according to the present invention, a mass ratio of the active ingredient to the first surfactant and the second surfactant (the active ingredient : a total of the first surfactant and the second surfactant) is 1 : 0.1 to 1 : 100.

The preparation according to the present invention includes the core-shell structure configured in accordance with the present invention.

The external medicine according to the present invention includes the core-shell structure configured in accordance with the present invention.

The tape preparation according to the present invention includes the core-shell structure configured in accordance with the present invention.

The cosmetic according to the present invention includes the core-shell structure configured in accordance with the present invention.

## EFFECT OF THE INVENTION

According to the present invention, it is possible to provide a core-shell structure, a preparation, an external medicine, a tape preparation, and a cosmetic that can achieve both improvement in transdermal absorption of an active ingredient and reduction in skin irritation at a high level.

## BRIEF DESCRIPTION OF DRAWINGS

[Fig. 1] Fig. 1 is a schematic sectional view showing a core-shell structure according to one embodiment of the present invention.

[Fig. 2] Fig. 2 is a view for illustrating a hydrophilic moiety and a hydrophobic moiety of a surfactant including an ester bond formed between an alcohol and a fatty acid.

[Fig. 3] Fig. 3 is a view for illustrating a hydrophilic moiety and a hydrophobic moiety of a surfactant including an amide bond formed between an alcohol and a fatty acid.

[Fig. 4] Fig. 4 is a schematic sectional view showing a tape preparation according to one embodiment of the present invention.

[Fig. 5] Fig. 5 is a schematic sectional view of a cell for a skin permeation test for a drug used in Test Example 1.

## MODES FOR CARRYING OUT THE INVENTION

Hereinafter, details of the present invention will be described.

[Core-shell structure]

The core-shell structure according to the present invention includes a core portion containing an active ingredient and a shell portion containing a surfactant.

In the present invention, the core portion and the shell portion may be connected by intermolecular force or the like to form an aggregate. However, from the viewpoint of further enhancing the transdermal absorption of the active ingredient, it is preferable that at least a part of the surface of the core portion be covered with the shell portion.

More specifically, it is preferable that 30% or more of the surface of the core portion be covered with the shell portion. The core portion is more preferably covered at 50% or more, still more preferably 70% or more, still even more preferably 85% or more, particularly preferably 95% or more, and most preferably 99% or more of the surface. However, the surface of the core portion may be completely covered with the shell portion. Because of the above-described configuration of the core-shell structure, the active ingredient contained in the core portion can be released into the body when the core-shell structure is applied, for example, to the skin.

In the present invention, the core portion is solid. Because the core portion is solid, the stability in the base described below can be further improved. In this case, a preparation having an S/O (Solid in Oil) structure can be formed by dispersing the core-shell structure in the base phase that is an oil phase.

As will be described below in the section on the production method, the core-shell structure according to the present invention is obtained by drying a W/O emulsion to remove the solvent (the aqueous solvent and the oil solvent). Therefore, the core portion is a solid (S in the S/O (Solid in Oil) structure). It is preferable that water be substantially completely removed by the step of drying the W/O emulsion. Specifically, the water content measured by, for example, the Karl Fischer method is preferably 5% by weight or less, more preferably 2% by weight or less, still more preferably 1% by weight or less, and particularly preferably 0.5% by weight or less. Therefore, the core-shell structure according to the present invention is different from the W/O emulsion.

In the present invention, the shell portion contains both a first surfactant having a melting point of less than 35°C and a second surfactant having a melting point of 35°C or more.

Because the core-shell structure according to the present invention includes the shell portion containing two surfactants having different melting points as described above, the core-shell structure can achieve both improvement in transdermal absorption of an active ingredient and reduction in skin irritation at a high level. When used in a tape preparation, the core-shell structure can effectively enhance both the adhesive strength and the holding strength.

In the present invention, the first surfactant and the second surfactant contained in the shell portion each have an HLB value of 4 to 14. In the present specification, the phrase "oo to oo" means "oo or more and oo or less". For example, the phrase "an HLB value of 4 to 14" means an HLB value of 4 or more and 14 or less.

The core-shell structure according to the present invention provides excellent transdermal absorption of the active ingredient because the HLB values of the first surfactant and the second surfactant contained in the shell portion are within the specific range as described above.

The reason for this fact can be explained as follows. When the HLB value of the surfactant contained in the shell portion is within the specific range described above, the skin barrier function can be reduced, so that a core-shell structure that provides excellent transdermal absorption can be obtained.

In the present invention, the first surfactant and the second surfactant each contain at least one selected from the group consisting of sorbitan fatty acid esters, glycerin fatty acid esters, propylene glycol fatty acid esters, and fatty acid alkanolamides. Therefore, the transdermal absorption can be effectively enhanced also from this point.

The core-shell structure according to the present invention can achieve both improvement in transdermal absorption of an active ingredient and reduction in skin irritation at a high level. Therefore, the core-shell structure can be suitably used in a preparation. In particular, the core-shell structure can be suitably used in the fields of external medicines, tape preparations, cosmetics, injections, and the like.

Hereinafter, an example of the core-shell structure according to the present invention will be described with reference to the drawings.

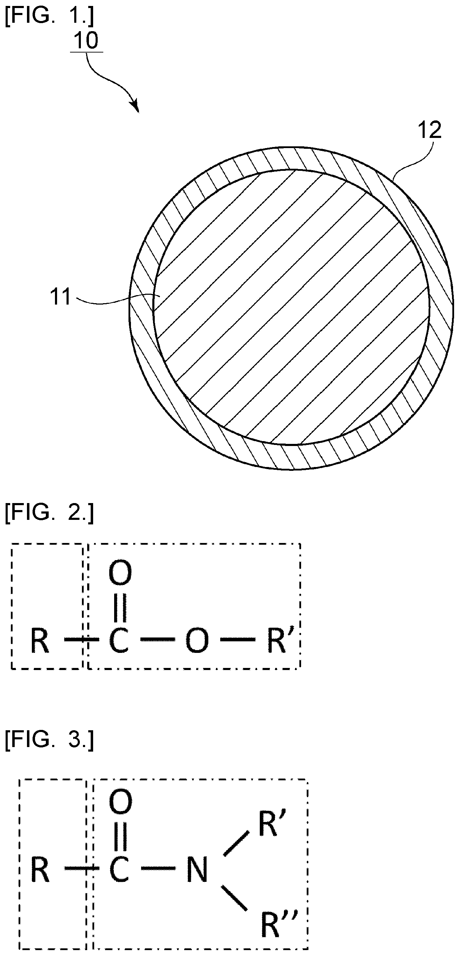

Fig. 1 is a schematic sectional view showing a core-shell structure according to one embodiment of the present invention.

As shown in Fig. 1, a core-shell structure 10 includes a core portion 11 and a shell portion 12. The surface of the core portion 11 is covered with the shell portion 12.

However, the shape of the core-shell structure according to the present invention is not limited to such a spherical particle shape. The core-shell structure according to the present invention may be a particle having, for example, a rod-like, cubic, lens-like, micellar, lamellar, hexagonal, bicellular, sponge-like, or sea urchin like shape, or may be amorphous. Thus, the shape of the core-shell structure according to the present invention is not particularly limited. However, as described above, it is preferable that at least a part of the surface of the core portion be covered with the shell portion.

The size of the core-shell structure according to the present invention is not particularly limited. From the viewpoint of further enhancing the transdermal absorption of the active ingredient, the average size of the core-shell structure can be preferably 1 nm to 100 $\mu$m.

In the present invention, the phrase "average size of the core-shell structure" means a number average size calculated by a dynamic light scattering method at the time of dispersion in a solvent (such as squalane).

Hereinafter, details of the core portion and the shell portion will be described.

(Core portion)

The core portion contains at least an active ingredient.

Specific examples of the active ingredient are not particularly limited, and include dementia therapeutic agents, antiepileptics, antidepressants, antiparkinsonian agents, antiallergic agents, anticancer agents, antidiabetic agents, antihypertensive agents, respiratory disease drugs, erectile dysfunction therapeutic agents, skin disease drugs, and local anesthetics. The active ingredients may be used singly or in combination of two or more kinds thereof.

More specific examples include memantine, donepezil, diphenhydramine, vardenafil, octreotide, rivastigmine, galantamine, nitroglycerin, lidocaine, fentanyl, male hormones, female hormones, nicotine, clomipramine, nalfurafine, metoprolol, fesoterodine, tandospirone, beraprost sodium, taltirelin, lurasidone, nefazodone, rifaximin, benidipine, doxazosin, nicardipine, formoterol, lomerizine, amlodipine, teriparatide, bucladesine, cromoglycic acid, lixisenatide, exenatide, liraglutide, lanreotide, glucagon, oxytocin, calcitonin, elcatonin, glatiramer, risedronate, diclofenac, ascorbic acid, and pharmaceutically acceptable salts thereof.

The pharmaceutically acceptable salts are not particularly limited, and acidic salts and basic salts can be used. Examples of the acidic salts include inorganic acid salts such as hydrochlorides, hydrobromates, sulfates, nitrates, and phosphates, and organic acid salts such as acetates, propionates, tartrates, fumarates, maleates, malates, citrates, methane sulfonates, benzene sulfonates, and para-toluenesulfonates. Examples of the basic salts include alkali metal salts such as sodium salts and potassium salts, and alkaline earth metal salts such as calcium salts and magnesium salts. Specific examples of the salt of the active ingredient include memantine hydrochloride, donepezil hydrochloride, rivastigmine tartrate, galantamine hydrobromide, clomipramine hydrochloride, diphenhydramine hydrochloride, nalfurafine hydrochloride, metoprolol tartrate, fesoterodine fumarate, vardenafil hydrochloride hydrate, nalfurafine hydrochloride, tandospirone citrate, beraprost sodium, lurasidone hydrochloride, nefazodone hydrochloride, benidipine hydrochloride, doxazosin mesilate, nicardipine hydrochloride, formoterol fumarate, lomerizine hydrochloride, and amlodipine besilate.

The active ingredient to be blended in a cosmetic is not particularly limited as long as the active ingredient needs to penetrate the skin. Examples of the active ingredient include vitamin ingredients such as vitamin C and vitamin E, moisturizing ingredients such as hyaluronic acid, ceramide, and collagen, skin-whitening ingredients such as tranexamic acid and arbutin, hair growth ingredients such as minoxidil, beauty ingredients such as fibroblast growth factor (FGF) and epidermal growth factor (EGF), and salts and derivatives thereof.

The active ingredient in the present invention preferably has low skin irritation. The phrase "low skin irritation" means skin irritation having a primary irritation index (P.I.I.) of 5 or less. The primary irritation index can be measured by the following method.

1. Production of preparation

An active ingredient is added and mixed to an ointment base, Plastibase (manufactured by Taisho Pharmaceutical Co., Ltd.) so as to be 4% by weight based on the total weight, and dispersed to produce a preparation.

2. Evaluation of skin irritation (evaluation of primary irritation index)

The dorsal skin of a rabbit is shaved with an electric clipper (with an electric shaver if necessary). On the dorsal healthy skin, two points in each of right and left sides of the dorsal midline, that is, four points in total are set to be an administration site. Next, the produced preparation is taken out with a spatula and spread evenly on a piece of lint having a size of 2 cm × 2 cm, and the piece of lint is attached to the administration site. The piece of lint is fixed by covering with a non-woven adhesive bandage (manufactured by Nichiban Co., Ltd., MESHPORE, No. 50). Then, the administration sites are altogether wrapped with gauze and enclosed by covering with an adhesive cloth elastic bandage (manufactured by Nichiban Co., Ltd., ELASTOPORE, No. 100). The enclosure is released 24 hours after the start of administration, and the administered samples are removed.

The skin reaction is visually observed with the naked eye 24 hours after the administration (30 minutes after the release of the enclosure and the removal of the administered samples). Then, furthermore, the skin reaction is visually observed with the naked eye 48 hours and 72 hours after the administration (30 minutes after the release of the enclosure and the removal of the administered samples) in the same manner. The skin reaction is evaluated in accordance with the Draize criteria shown in Table 1 below.

[Table 1]

| Degree of skin reaction | Score |
| --- | --- |
| Erythema/eschar formation | |
| No erythema | 0 |
| Very slight erythema (in barely detectable degree) | 1 |
| Apparent erythema | 2 |
| Moderate to severe erythema | 3 |
| Severe erythema with deep red color and slight eschar formation (with deep injury) | 4 |
| Edema formation | |
| No edema | 0 |

(continued)

| Edema formation | |
|---|---|
| Very slight edema (in barely detectable degree) | 1 |
| Apparent edema (well defined from surrounding area) | 2 |
| Moderate edema (raised by approximately 1 mm) | 3 |
| Severe edema (raised by 1 mm or more and extending to surrounding area) | 4 |

Specifically, for each administered sample at each time of observation, the individual score of the skin reaction at the administration site of each rabbit (the total of scores of erythema/eschar formation and edema formation) is calculated. Then, the primary irritation index (P.I.I.) is calculated from the individual scores at 24 hours and 72 hours after the administration (the score at 48 hours after the administration is not added). Specifically, the primary irritation index is calculated using Formulae (1) and (2) described below.

$$\text{Average score of each administration site} = (\text{total of individual scores at 24 hours and 72 hours after administration})/2 \ldots \text{Formula (1)}$$

$$\text{Primary irritation index (P.I.I.)} = (\text{total of average scores of administration sites})/(3\ (\text{rabbits})) \ldots \text{Formula (2)}$$

The primary irritation index measured by the above-described method is preferably 2 or less, and more preferably 1 or less.

Examples of the active ingredient having low skin irritation include loxoprofen sodium dihydrate (P.I.I = 0.3), rivastigmine (P.I.I. = 0.5), donepezil (P.I.I. = 0.5), and memantine hydrochloride (P.I.I. = 2.5).

The active ingredient is preferably hydrophilic. When the active ingredient is a hydrophilic drug, a drug that needs to have systemic or local effect is usually used.

The active ingredient is preferably a transdermally absorbable drug. The active ingredient is not particularly limited, and is preferably a compound having an octanol/water partition coefficient of -2 to 6. In this case, the transdermal absorption of the active ingredient is further improved. From the viewpoint of further improving the transdermal absorption of the active ingredient, the octanol/water partition coefficient is preferably -1 or more, and more preferably 0 or more. The octanol/water partition coefficient of the active ingredient is preferably 4 or less, and more preferably 1 or less. When the octanol/water partition coefficient of the active ingredient is the above-described upper limit or less, the transdermal absorption of the active ingredient is further improved.

In the present invention, the octanol/water partition coefficient is determined from the concentration of the active ingredient in each phase by adding the active ingredient to octanol and an aqueous buffer solution having a pH of 7 in a flask and then shaking the flask. Specifically, the octanol/water partition coefficient can be determined by calculating using the formula: octanol/water partition coefficient = $\text{Log}_{10}$(concentration in octanol phase/concentration in aqueous phase).

The amount of the active ingredient contained in the core-shell structure depends on the kind of the active ingredient, but for example, the raw material weight is preferably 1% by weight to 70% by weight, and more preferably 5% by weight to 70% by weight. The raw material weight is a value based on the total weight of all the raw materials contained in the core-shell structure.

The core portion may contain two or more kinds of active ingredients as the active ingredient, if necessary.

The molecular weight of the active ingredient is not particularly limited. The molecular weight of the active ingredient is preferably 250 g/mol or more and more preferably 300 g/mol or more, and preferably 7,500 g/mol or less, more preferably 6,500 g/mol or less, and still more preferably 1,500 g/mol or less.

(Shell portion)

In the present invention, the shell portion contains both the first surfactant having a melting point of less than 35°C and the second surfactant having a melting point of 35°C or more.

The melting point of the first surfactant is preferably -60°C or more and more preferably 0°C or more, and preferably 34°C or less and more preferably 30°C or less. When the melting point of the first surfactant is within the above-described range, it is possible to achieve both improvement in the transdermal absorption of the active ingredient and reduction in the skin irritation at a further high level.

The melting point of the second surfactant is preferably 35°C or more and more preferably 40°C or more, and preferably 70°C or less and more preferably 60°C or less. When the melting point of the second surfactant is within the above-described range, it is possible to achieve both improvement in the transdermal absorption of the active ingredient and reduction in the skin irritation at a further high level.

The melting point in the present invention is a value determined from the endothermic peak in measurement by differential scanning calorimetry (DSC) described below.

[Measurement conditions]

Sample amount: 10 mg
Temperature condition: holding at -150°C for 20 minutes and heating to 75°C at a rate of 6°C/min after the holding.
Measurement atmosphere: under nitrogen atmosphere

As the measuring device, for example, DSC6220 (manufactured by Hitachi High-Tech Science Corporation) can be used.

In the present invention, the first surfactant and the second surfactant each have an HLB value of 4 to 14.

The HLB (abbreviation of Hydrophile Lipophile Balance) value in the present invention is an index of the hydrophilicity or lipophilicity of an emulsifier, and is a value of 0 to 20. The smaller the HLB value is, the more lipophilic the emulsifier is.

In the present invention, the HLB value is calculated by the Griffin formula described below.

$$\text{HLB value} = 20 \times \{(\text{molecular weight of hydrophilic moiety})/(\text{total molecular weight})\}$$

The weighted average value of the HLB value can be calculated using, for example, the following formula.

When surfactants having HLB values of A, B, and C have weights of x, y, and z respectively, the formula for calculation of the weighted average value is $(xA + yB + zC) \div (x + y + z)$.

The first surfactant and the second surfactant each have an HLB value or, when the first surfactant and the second surfactant each include a plurality of surfactants, a weighted average value of the HLB values of 4 or more and 14 or less, and more preferably 5 or more and 12 or less.

In the present invention, the first surfactant and the second surfactant each preferably have at least one of a saturated hydrocarbon group such as an alkyl group or an unsaturated hydrocarbon group such as an alkenyl group or an alkynyl group.

Furthermore, the first surfactant and the second surfactant each preferably have a saturated hydrocarbon group having 7 to 15 carbon atoms or an unsaturated hydrocarbon group having 7 to 17 carbon atoms. When the number of carbon atoms in the hydrocarbon group is within the above-described specific range, the release property of the active ingredient from a particle is further improved in the body. Therefore, a core-shell structure can be obtained that provides further excellent transdermal absorption.

The number of carbon atoms in the saturated hydrocarbon group is preferably 5 or more and 15 or less, and more preferably 7 or more and 11 or less. When the number of carbon atoms in the saturated hydrocarbon group is the above-described lower limit or more, the coatability of the surface of the core portion by the shell portion is further improved. Therefore, a core-shell structure can be obtained that provides further excellent transdermal absorption. When the number of carbon atoms in the saturated hydrocarbon group is the above-described upper limit or less, the release property of the active ingredient from the core-shell structure is further improved in the body, and therefore, a core-shell structure can be obtained that provides further excellent transdermal absorption.

The number of carbon atoms in the unsaturated hydrocarbon group is preferably 5 or more and 17 or less, more preferably 7 or more and 13 or less, and still more preferably 7 or more and 11 or less. When the number of carbon atoms in the unsaturated hydrocarbon group is the above-described lower limit or more, the coatability of the surface of the core portion by the shell portion is further improved. Therefore, a core-shell structure can be obtained that provides further excellent transdermal absorption. When the number of carbon atoms in the unsaturated hydrocarbon group is the above-described upper limit or less, the release property of the active ingredient from the core-shell structure is further improved in the body, and therefore, a core-shell structure can be obtained that provides further excellent transdermal absorption.

When the first surfactant and the second surfactant each include a plurality of hydrocarbon groups, the hydrocarbon group included at the largest proportion in the surfactant is regarded as the hydrocarbon group of the surfactant in the present invention.

In particular, when the surfactant includes a plurality of hydrocarbon groups having different numbers of carbon atoms, the number of carbon atoms in the hydrocarbon group included at the largest proportion in the surfactant is regarded as the number of carbon atoms in the hydrocarbon group of the surfactant in the present invention.

For example, when the surfactant is specifically a coconut oil fatty acid ester, the saturated hydrocarbon group having 11 carbon atoms is included at the largest proportion in the surfactant. Therefore, the hydrocarbon group of the coconut oil fatty acid ester is the saturated hydrocarbon, and the number of carbon atoms in the hydrocarbon group is 11.

When the first surfactant and the second surfactant each include a plurality of surfactants, the number of carbon atoms in the hydrocarbon group included at the largest proportion in the plurality of surfactants is regarded as the number of carbon atoms in the hydrocarbon group of each of the first surfactant and the second surfactant.

In the present invention, the number of carbon atoms in the hydrocarbon group included in the first surfactant is preferably 5 or more and more preferably 6 or more, and preferably 8 or less and more preferably 7 or less. When the number of carbon atoms in the hydrocarbon group included in the first surfactant is the above-described lower limit or more, the coatability of the surface of the core portion by the shell portion is further improved. Therefore, a core-shell structure can be obtained that provides further excellent transdermal absorption. When the number of carbon atoms in the hydrocarbon group included in the first surfactant is the above-described upper limit or less, the influence of the shell portion on the skin tissue is further reduced, and therefore, a core-shell structure can be obtained that is further excellent in skin irritation.

In the present invention, the number of carbon atoms in the hydrocarbon group included in the second surfactant is preferably 9 or more, and preferably 17 or less and more preferably 15 or less. When the number of carbon atoms in the hydrocarbon group included in the second surfactant is the above-described lower limit or more, the coatability of the surface of the core portion by the shell portion is further improved. Therefore, a core-shell structure can be obtained that provides further excellent transdermal absorption. When the number of carbon atoms in the hydrocarbon group included in the second surfactant is the above-described upper limit or less, the influence of the shell portion on the skin tissue is further reduced, and therefore, a core-shell structure can be obtained that is further excellent in skin irritation.

In the present invention, the molecular weight of the hydrophilic moiety in each of the first surfactant and the second surfactant is preferably 100 g/mol or more and 350 g/mol or less, more preferably 100 g/mol or more and 300 g/mol or less, and still more preferably 100 g/mol or more and 200 g/mol or less. When the molecular weight of the hydrophilic moiety in the surfactant is the above-described lower limit or more, the coatability of the core portion by the shell portion is further improved. Therefore, a core-shell structure can be obtained that provides further enhanced transdermal absorption. When the molecular weight of the hydrophilic moiety in the surfactant is the above-described upper limit or less, the release property of the active ingredient from a particle in the body is further improved. Therefore, a core-shell structure can be obtained that provides further enhanced transdermal absorption.

The phrase "hydrophilic moiety in the surfactant" means a moiety obtained by removing the hydrocarbon group in the constituent fatty acid from the entire surfactant molecule. For example, in the case of sorbitan monooleate, the molecular weight of the entire surfactant molecule is 428.6 g/mol, and the molecular weight of the hydrocarbon group in monooleic acid, which is a constituent fatty acid, is 237.4 g/mol. Therefore, the molecular weight of the hydrophilic moiety in the surfactant is calculated as 191.2 g/mol by subtracting the molecular weight of the hydrocarbon group in the constituent fatty acid from the molecular weight of the entire surfactant molecule.

Furthermore, the first surfactant and the second surfactant each preferably include an ester bond or an amide bond formed between an alcohol and a fatty acid. In this case, the molecular weight of the alcohol is preferably 70 g/mol or more and more preferably 80 g/mol or more, and preferably 330 g/mol or less, more preferably 300 g/mol or less, still more preferably 250 g/mol or less, and particularly preferably 200 g/mol or less.

When the molecular weight of the alcohol is the above-described lower limit or more, the coatability of the core portion by the shell portion is further improved. Therefore, a core-shell structure can be obtained that provides further enhanced transdermal absorption. When the molecular weight of the alcohol is the above-described upper limit or less, the release property of the active ingredient from a particle in the body is further improved. Therefore, a core-shell structure can be obtained that provides further enhanced transdermal absorption. Note that the amide bond formed between an alcohol and a fatty acid is an amide bond formed between an alkanolamine and a fatty acid. Therefore, in this case, the phrase "molecular weight of the alcohol" means the molecular weight of the alkanolamine.

Hereinafter, a hydrophilic moiety and a hydrophobic moiety of a surfactant including an ester bond formed between an alcohol and a fatty acid will be described with reference to Fig. 2. As shown in Fig. 2, in the case of an ester bond formed between an alcohol and a fatty acid, the moiety surrounded by the broken line in Fig. 2 is the hydrophobic moiety. The number of carbon atoms in the hydrocarbon group is the number of carbon atoms included in R in the hydrophobic moiety. Therefore, even when R in the hydrophobic moiety includes an ether bond or the like, the total number of carbon atoms included only in R in the hydrophobic moiety is determined as the number of carbon atoms. The moiety surrounded by the alternate long and short dash line in Fig. 2 is the hydrophilic moiety. R'O in the hydrophilic moiety represents the alcohol moiety. Therefore, the original alcohol is represented by R'OH. In this case, the molecular weight of the alcohol is the molecular weight of R'OH.

Hereinafter, a hydrophilic moiety and a hydrophobic moiety of a surfactant including an amide bond formed between an alcohol and a fatty acid will be described with reference to Fig. 3. As shown in Fig. 3, in the case of an amide bond formed between an alcohol and a fatty acid, the moiety surrounded by the broken line in Fig. 3 is the hydrophobic moiety. The number of carbon atoms in the hydrocarbon group is the number of carbon atoms included in R in the hydrophobic moiety. Therefore, even when R in the hydrophobic moiety includes an ether bond or the like, the total number of carbon atoms included only in R in the hydrophobic moiety is determined as the number of carbon atoms. The moiety surrounded by the alternate long and short dash line in Fig. 3 is the hydrophilic moiety. R'R"N in the hydrophilic moiety represents the alcohol moiety. Therefore, the original alcohol is represented by R'R"NH. In this case, the molecular weight of the alcohol is the molecular weight of R'R"NH.

The first surfactant and the second surfactant each contain at least one selected from the group consisting of sorbitan fatty acid esters, glycerin fatty acid esters, propylene glycol fatty acid esters, and fatty acid alkanolamides.

In particular, from the viewpoint of achieving both transdermal absorption of the active ingredient and reduction of the irritation at a further high level, the first surfactant and the second surfactant each preferably contain at least one selected from the group consisting of sorbitan fatty acid esters, glycerin fatty acid esters, and propylene glycol fatty acid esters.

The sorbitan fatty acid ester in the present invention is not particularly limited, and examples thereof include an ester of sorbitan and a fatty acid.

Examples of the fatty acid include caproic acid, caprylic acid, capric acid, lauric acid, myristic acid, palmitic acid, stearic acid, behenic acid, undecylenic acid, ricinoleic acid, oleic acid, linoleic acid, linolenic acid, ricinoleic acid, erucic acid, beef tallow, lard, coconut oil, palm oil, palm kernel oil, olive oil, rapeseed oil, rice bran oil, soy oil, and castor oil.

Specifically, from the viewpoint of further enhancing the transdermal absorption of the active ingredient, preferable examples of the sorbitan fatty acid ester include sorbitan monostearate (NIKKOL SO-10MV, manufactured by NIPPON SURFACTANT INDUSTRIES CO., LTD.), sorbitan trioleate (NIKKOL SO-30V, manufactured by NIPPON SURFACTANT INDUSTRIES CO., LTD.), sorbitan sesquioleate (NIKKOL SO-15MV, manufactured by NIPPON SURFACTANT INDUSTRIES CO., LTD.), sorbitan monooleate (NIKKOL SO-10V, manufactured by NIPPON SURFACTANT INDUSTRIES CO., LTD.), sorbitan monolaurate (NIKKOL SL-10, manufactured by NIPPON SURFACTANT INDUSTRIES CO., LTD.), coconut oil fatty acid sorbitan (EMALEX SPC-10, manufactured by Nihon Emulsion Co., Ltd.), sorbitan laurate (RIKEMAL L-250A, manufactured by RIKEN VITAMIN CO., LTD.), sorbitan monopalmitate (product name "NIKKOL SP-10V", manufactured by NIPPON SURFACTANT INDUSTRIES CO., LTD.), and sorbitan monomyristate (product name "NONION MP-30R", manufactured by NOF CORPORATION).

The glycerin fatty acid ester in the present invention is not particularly limited, and examples thereof include an ester of glycerin and a fatty acid.

The glycerin may be polyglycerin. The polymerization degree n of polyglycerin is not particularly limited, and is preferably 5 or less, more preferably 4 or less, and still more preferably 3 or less. Monoglycerin, diglycerin, and triglycerin are particularly preferable as the glycerin.

The glycerin fatty acid ester is preferably at least one selected from the group consisting of monoglycerin fatty acid esters, diglycerin fatty acid esters, and triglycerin fatty acid esters. In this case, the transdermal absorption of the active ingredient can be further enhanced.

Examples of the fatty acid include caproic acid, caprylic acid, capric acid, lauric acid, myristic acid, palmitic acid, stearic acid, behenic acid, undecylenic acid, ricinoleic acid, oleic acid, linoleic acid, linolenic acid, ricinoleic acid, erucic acid, beef tallow, lard, coconut oil, palm oil, palm kernel oil, olive oil, rapeseed oil, rice bran oil, soy oil, and castor oil.

Specifically, from the viewpoint of further enhancing the transdermal absorption of the active ingredient, preferable examples of the glycerin fatty acid ester include diglyceryl monostearate (NIKKOL DGMS, manufactured by NIPPON SURFACTANT INDUSTRIES CO., LTD.), glyceryl monostearate (NIKKOL MGS-BMV, manufactured by NIPPON SURFACTANT INDUSTRIES CO., LTD.), glyceryl monostearate (NIKKOL MGS-AMV, manufactured by NIPPON SURFACTANT INDUSTRIES CO., LTD.), glyceryl monostearate (NIKKOL MGS-DEXV, manufactured by NIPPON SURFACTANT INDUSTRIES CO., LTD.), glyceryl monostearate (NIKKOL MGS-ASEV, manufactured by NIPPON SURFACTANT INDUSTRIES CO., LTD.), glyceryl monostearate (NIKKOL MGS-BSEV, manufactured by NIPPON SURFACTANT INDUSTRIES CO., LTD.), glyceryl myristate (MGM, manufactured by NIPPON SURFACTANT INDUSTRIES CO., LTD.), caprylic/capric triglyceride (NIKKOL Triester F-810, manufactured by NIPPON SURFACTANT INDUSTRIES CO., LTD.), glyceryl monooleate (NIKKOL MGO, manufactured by NIPPON SURFACTANT INDUSTRIES CO., LTD.), glyceryl monooleate (Capmul GMO-50, manufactured by ABITEC), glyceryl monoolivate (NIKKOL MGOL-70, manufactured by NIPPON SURFACTANT INDUSTRIES CO., LTD.), diglyceryl monooleate (NIKKOL DGMO-CV, manufactured by NIPPON SURFACTANT INDUSTRIES CO., LTD.), diglyceryl monooleate (NIKKOL DGMO-90V, manufactured by NIPPON SURFACTANT INDUSTRIES CO., LTD.), glyceryl monocaprylate (Sunsoft No. 700P-2-C, manufactured by Taiyo Kagaku Co., Ltd.), glyceryl monocaprylate (Capmul 808G, manufactured by ABITEC), glyceryl monocaprylate (Capmul MCM C8, manufactured by ABITEC), glyceryl monocaprate (Sunsoft No. 760-C, manufactured by Taiyo Kagaku Co., Ltd.), glyceryl caprate (Capmul MCM C10, manufactured by ABITEC), glyceryl caprylate/caprate (Capmul MCM, manufactured by ABITEC), glyceryl caprylate/caprate (Capmul 471, manufactured by ABITEC), capric acid mono/diglyceride (Sunsoft No. 707-C, manufactured by Taiyo Kagaku Co., Ltd.), capric acid diglyceride (Sunfat GDC-S, manufactured by Taiyo Kagaku Co., Ltd.), glyceryl monolaurate (Sunsoft No. 750-C, manufactured by Taiyo Kagaku Co., Ltd.), glyceryl monoundecylenate (NIKKOL MGU, manufactured by NIPPON SURFACTANT INDUSTRIES CO., LTD.), glyceryl monopalmitate (product name "Monopalmitin", manufactured by AccuStandard), and glyceryl monomyristate (product name "NIKKOL MGM", manufactured by NIPPON SURFACTANT INDUSTRIES CO., LTD.).

More preferable examples of the glycerin fatty acid ester include glyceryl monooleate (NIKKOL MGO), glyceryl monooleate (Capmul GMO-50, manufactured by ABITEC), glyceryl monoolivate (NIKKOL MGOL-70, manufactured by NIPPON SURFACTANT INDUSTRIES CO., LTD.), diglyceryl monooleate (NIKKOL DGMO-CV, manufactured by NIPPON SURFACTANT INDUSTRIES CO., LTD.), diglyceryl monooleate (NIKKOL DGMO-90V, manufactured by NIPPON SURFACTANT INDUSTRIES CO., LTD.), glyceryl monocaprylate (Sunsoft No. 700P-2-C, manufactured by Taiyo Kagaku Co., Ltd.), glyceryl monocaprylate (Capmul 808G, manufactured by ABITEC), glyceryl monocaprylate (Capmul MCM C8, manufactured by ABITEC), glyceryl monocaprate (Sunsoft No. 760-C, manufactured by Taiyo Kagaku Co., Ltd.), glyceryl caprate (Capmul MCM C10, manufactured by ABITEC), glyceryl caprylate/caprate (Capmul MCM, manufactured by ABITEC), glyceryl caprylate/caprate (Capmul 471, manufactured by ABITEC), capric acid mono/diglyceride (Sunsoft No. 707-C, manufactured by Taiyo Kagaku Co., Ltd.), capric acid diglyceride (Sunfat GDC-S, manufactured by Taiyo Kagaku Co., Ltd.), glyceryl monolaurate (Sunsoft No. 750-C, manufactured by Taiyo Kagaku Co., Ltd.), glyceryl monoundecylenate (NIKKOL MGU, manufactured by NIPPON SURFACTANT INDUSTRIES CO., LTD.), glyceryl monopalmitate (product name "Monopalmitin", manufactured by AccuStandard), and glyceryl monomyristate (product name "NIKKOL MGM", manufactured by NIPPON SURFACTANT INDUSTRIES CO., LTD.).

The propylene glycol fatty acid ester in the present invention is not particularly limited, and examples thereof include an ester of propylene glycol and a fatty acid.

Examples of the fatty acid include caproic acid, caprylic acid, capric acid, lauric acid, myristic acid, palmitic acid, stearic acid, behenic acid, undecylenic acid, ricinoleic acid, oleic acid, linoleic acid, linolenic acid, ricinoleic acid, erucic acid, beef tallow, lard, coconut oil, palm oil, palm kernel oil, olive oil, rapeseed oil, rice bran oil, soy oil, and castor oil.

Specifically, from the viewpoint of further enhancing the transdermal absorption of the active ingredient, preferable examples of the propylene glycol fatty acid ester include propylene glycol monostearate (RIKEMAL PS-100, manufactured by RIKEN VITAMIN CO., LTD.), propylene glycol monostearate (NIKKOL PMS-1CV, manufactured by NIPPON SURFACTANT INDUSTRIES CO., LTD.), propylene glycol diisostearate (EMALEX PG-di-IS, manufactured by Nihon Emulsion Co., Ltd.), propylene glycol distearate (EMALEX PG-di-S, manufactured by Nihon Emulsion Co., Ltd.), propylene glycol monolaurate (RIKEMAL PL-100, manufactured by RIKEN VITAMIN CO., LTD.), propylene glycol monooleate (RIKEMAL PO-100, manufactured by RIKEN VITAMIN CO., LTD.), propylene glycol dioleate (EMALEX PG-di-O, manufactured by Nihon Emulsion Co., Ltd.), propylene glycol dicaprylate (NIKKOL SEFSOL-228, manufactured by NIPPON SURFACTANT INDUSTRIES CO., LTD.), propylene glycol dilaurate (EMALEX PG-M-L, manufactured by Nihon Emulsion Co., Ltd.), and propylene glycol monocaprylate (product name "NIKKOL SEFSOL-218", manufactured by NIPPON SURFACTANT INDUSTRIES CO., LTD.).

The fatty acid alkanolamide in the present invention has a structure in which R-CO and two -$CH_2CH_2OH$ are bonded to N at the center, and is represented by the chemical formula of R-$CON(CH_2CH_2OH)_2$.

Specific examples of the fatty acid alkanolamide include oleic acid diethanolamide, lauric acid diethanolamide, lauric acid monoisopropanolamide, stearic acid diethanolamide, stearic acid monoethanolamide, stearic acid monoisopropanolamide, lauric acid/myristic acid diethanolamide, palmitic acid monoethanolamide, coconut oil fatty acid diethanolamide, coconut oil fatty acid monoisopropanolamide, coconut oil fatty acid N-methylethanolamide, coconut oil fatty acid monoethanolamide, and palm kernel oil fatty acid diethanolamide. From the viewpoint of further enhancing the skin penetration, the fatty acid alkanolamide is preferably a diethanolamide such as oleic acid diethanolamide, lauric acid diethanolamide, or coconut oil fatty acid diethanolamide.

The first surfactant and the second surfactant each may further contain a surfactant, other than a sorbitan fatty acid ester, a glycerin fatty acid ester, a propylene glycol fatty acid ester, or a fatty acid alkanolamide. The surfactant can be appropriately selected according to its use. For example, a surfactant can be selected widely from the surfactants that can be used as pharmaceuticals and cosmetics. Furthermore, a plurality of surfactants may be used in combination.

The surfactant other than a sorbitan fatty acid ester, a glycerin fatty acid ester, a propylene glycol fatty acid ester, or a fatty acid alkanolamide may be any of a nonionic surfactant, an anionic surfactant, a cationic surfactant, and an amphoteric surfactant.

The nonionic surfactant is not particularly limited, and examples thereof include fatty acid esters, fatty alcohol ethoxylates, polyoxyethylene alkyl phenyl ethers, alkyl glycosides, polyoxyethylene castor oil, and hydrogenated castor oil.

The fatty acid ester is not particularly limited, and examples thereof include esters of at least one of glycerin, polyglycerin, polyoxyethylene glycerin, polyoxyethylene, sorbitan, propylene glycol, polyoxyethylene sorbit, or the like and a fatty acid such as caproic acid, caprylic acid, capric acid, lauric acid, myristic acid, palmitic acid, stearic acid, behenic acid, undecylenic acid, ricinoleic acid, oleic acid, linoleic acid, linolenic acid, ricinoleic acid, erucic acid, beef tallow, lard, coconut oil, palm oil, palm kernel oil, olive oil, rapeseed oil, rice bran oil, soy oil, or castor oil.

Examples of the anionic surfactant include alkyl sulfate salts, polyoxyethylene alkyl ether sulfate salts, alkylbenzene sulfonate salts, fatty acid salts, and phosphate salts.

Examples of the cationic surfactant include alkyltrimethylammonium salts, dialkyldimethylammonium salts, alkyldimethylbenzylammonium salts, and amine salts.

Examples of the amphoteric surfactant include alkylamino fatty acid salts, alkyl betaines, and alkyl amine oxides.

The surfactant other than a sorbitan fatty acid ester, a glycerin fatty acid ester, a propylene glycol fatty acid ester, or a fatty acid alkanolamide is particularly preferably a sucrose fatty acid ester, a polyoxyethylene glycerin fatty acid ester, a sorbitan fatty acid ester, a polyoxyethylene sorbit fatty acid ester, polyoxyethylene castor oil, or hydrogenated castor oil.

The surfactant other than a sorbitan fatty acid ester, a glycerin fatty acid ester, a propylene glycol fatty acid ester, or a fatty acid alkanolamide may have a hydrocarbon chain such as an alkyl chain, an alkenyl chain, or an alkynyl chain.

In the present invention, the mass ratio of the first surfactant to the second surfactant (the first surfactant : the second surfactant) is preferably 10 : 90 to 80 : 20. The mass ratio is more preferably 20 : 80 to 60 : 40, still more preferably 20 : 80 to 50 : 50, and particularly preferably 20 : 80 to 40 : 60. When the mass ratio is within the above-described specific range, it is possible to achieve both improvement in the transdermal absorption of the active ingredient and reduction in the skin irritation at a further high level.

In the present invention, the contents of the first surfactant and the second surfactant contained in the core-shell structure can be appropriately set within the range in which an effect of the present invention are exhibited.

However, the mass ratio of the active ingredient to the first surfactant and the second surfactant (the active ingredient : the total of the first surfactant and the second surfactant) is preferably 1 : 0.1 to 1 : 100, and more preferably 1 : 0.5 to 1 : 100. In this case, the transdermal absorption of the active ingredient in the core-shell structure and the preparation including the core-shell structure can be further enhanced. From the viewpoint of further enhancing the transdermal absorption of the active ingredient, the mass ratio is more preferably 1 : 0.5 to 1 : 50, and particularly preferably 1 : 0.5 to 1 : 30.

Furthermore, in the present invention, the mass ratio may be 1 : 0.5 to 1 : 2. In a tape preparation, as the content of the active ingredient is increased, the dispersibility of the active ingredient in the tape preparation generally tends to be deteriorated. However, in the present invention, the dispersibility in a tape preparation can be further improved even if the content of the active ingredient is large because the surfactant having the above-described HLB value is used.

(Another additive ingredient)

The core-shell structure may further contain at least one ingredient, other than the active ingredient, the first surfactant, or the second surfactant. The ingredient is not particularly limited, and examples thereof include stabilizing agents, transdermal absorption promoting agents, skin irritation-reducing agents, preservatives, and analgesics.

The stabilizing agent has an effect of stabilizing the particle structure. In addition, the stabilizing agent has a role of preventing unintended premature collapse of the particle structure and further enhancing the sustained release effect of the active ingredient.

The stabilizing agent is not particularly limited, and examples thereof include polysaccharides, proteins, and hydrophilic polymer materials. The core-shell structure may contain one stabilizing agent or two or more kinds of stabilizing agents. The content of the stabilizing agent can be appropriately set according to the kind of the stabilizing agent. For example, the stabilizing agent can be blended so that the weight ratio of the active ingredient to the stabilizing agent (the active ingredient : the stabilizing agent) is 1 : 0.1 to 1 : 10.

The transdermal absorption promoting agent is not particularly limited, and examples thereof include higher alcohols, N-acylsarcosine and its salts, higher monocarboxylic acids, higher monocarboxylic acid esters, aromatic monoterpene fatty acid esters, divalent carboxylic acids having 2 to 10 carbon atoms and salts of the divalent carboxylic acids, polyoxyethylene alkyl ether phosphoric acid esters and their salts, lactic acid, lactic acid esters, and citric acid. The core-shell structure may contain one transdermal absorption promoting agent or two or more kinds of transdermal absorption promoting agents. The content of the transdermal absorption promoting agent can be appropriately set according to the kind of the transdermal absorption promoting agent. For example, the transdermal absorption promoting agent can be blended so that the weight ratio of the active ingredient to the transdermal absorption promoting agent (the active ingredient : the transdermal absorption promoting agent) is 1 : 0.01 to 1 : 50.

The skin irritation-reducing agent is not particularly limited, and examples thereof include hydroquinone glycosides, pantethine, tranexamic acid, lecithin, titanium oxide, aluminum hydroxide, sodium nitrite, sodium hydrogen nitrite, soybean lecithin, methionine, glycyrrhetinic acid, dibutylhydroxytoluene (BHT), butylated hydroxyanisole (BHA), vitamin E and its derivatives, vitamin C and its derivatives, benzotriazole, propyl gallate, and mercaptobenzimidazole. The core-shell structure may contain one skin irritation-reducing agent or two or more kinds of skin irritation-reducing agents. The content ratio of the skin irritation-reducing agent can be appropriately set according to the kind of the skin irritation-reducing agent. The skin irritation-reducing agent can be blended, for example, so as to be 0.1% by weight to 50% by weight based on the entire core-shell structure.

The preservative is not particularly limited, and examples thereof include methyl parahydroxybenzoate, propyl parahydroxybenzoate, phenoxyethanol, and thymol. The content ratio of the preservative in the core portion can be appropriately set according to the kind of the preservative. The preservative can be blended, for example, so as to be 0.01% by weight to 10% by weight based on the entire core-shell structure. The core-shell structure may contain one preservative or two or more kinds of preservatives.

The analgesic is not particularly limited, and examples thereof include local anesthetics, such as procaine, tetracaine, lidocaine, dibucaine, and prilocaine, and their salts. The core-shell structure may contain one analgesic or two or more kinds of analgesics. The content ratio of the analgesic in the core-shell structure can be appropriately set according to the kind of the analgesic. The analgesic can be blended, for example, so as to be 0.1% by weight to 30% by weight based on the entire core-shell structure.

[Preparation]

The preparation according to the present invention includes at least the above-described core-shell structure. Since the preparation according to the present invention includes at least the core-shell structure, the preparation can achieve both improvement in transdermal absorption of an active ingredient and reduction in skin irritation at a high level.

The content ratio of the core-shell structure in the preparation is not particularly limited. In the case of an adhesive preparation, an ointment, a cream, or a gel, the content ratio is preferably 10% by mass or more and 70% by mass or less, and more preferably 20% by mass or more and 50% by mass or less.

The preparation according to the present invention can be used for a wide range of purposes intended for transdermal absorption and transmucosal absorption depending on the kind of the active ingredient. For example, the preparation can be used in an external medicine such as a skin external medicine, an eye drop, a nasal drop, a suppository, or an oral medicine, a cosmetic, or an injection.

The preparation according to the present invention is usually sustained for one day to one week although the period is not restrictive. In a preferable aspect, the preparation is used so as to be applied once per day to per week.

When the preparation according to the present invention is an external preparation, the target disease depends on the kind of the active ingredient.

The preparation according to the present invention can be used, without particular limitation, as a tape preparation such as a plaster (reservoir type tape preparation, matrix type tape preparation, or the like), an adhesive preparation such as a poultice, a patch, or a microneedle, an external liquid preparation such as an ointment, a liniment, or a lotion, a spray such as an external aerosol or a pump spray preparation, a cream, a gel, an eye drop, an eye ointment, a nasal drop, a suppository, a semisolid preparation for rectal, an enema agent, an oral agent, or an injection.

The preparation according to the present invention preferably has a water content of 20% by mass or less, and more preferably contains substantially no water. At such a water content, the core-shell structure can have a further enhanced shape retaining property. By a combined effect with the shape retaining property inherent in the core-shell structure, it is possible to further suppress leakage of the active ingredient from the core-shell structure and further suppress crystallization of the active ingredient. As a result, further high transdermal absorption can be exhibited. From this viewpoint, the preparation according to the present invention is preferably used as a preparation having a water content adjusted to 20% by mass or less. The preparation is more preferably used as a preparation containing substantially no water. The preparation according to the present invention is preferably used as, for example, a plaster, a patch, an ointment, or a gel.

(Base phase)

The preparation according to the present invention may include a base phase, and the base phase may include the core-shell structure. At this time, the core-shell structure is preferably dispersed or dissolved in the base phase.

The base is not particularly limited, and can be widely selected particularly from the bases that can be used as pharmaceuticals such as external medicines and as cosmetics.

As described above, the core-shell structure according to the present invention has a solid core portion. Therefore, when the base phase is an oil phase, an S/O (Solid in Oil) preparation can be formed by dispersing the core-shell structure in the base phase that is an oil phase. The S/O preparation can be obtained, for example, by dispersing the particles obtained by the production method described below in an oil phase.

The formed S/O (Solid in Oil) preparation is, for example, applied to a substrate to improve the transparency of the resulting coated sheet. Furthermore, in the formed S/O (Solid in Oil) preparation, for example, the diffraction pattern of the active ingredient measured by X-ray diffraction is different from the diffraction pattern of the single original active ingredient. When compared to the diffraction pattern of the coated sheet coated with the single active ingredient, the diffraction pattern of the coated sheet coated with the S/O preparation shows at least one of a change in the peak position, a change in the shape, or a decrease in the peak intensity. In particular, as for the decrease in the peak intensity in the X-ray diffraction spectrum, the peak intensity of the active ingredient is decreased to less than the peak intensity of the single original active ingredient. In this case, the peak of the active ingredient may disappear due to the decrease.

The base can be appropriately selected, according to the purpose of use and the like, from those suitable for dispersing or dissolving the core-shell structure, and is not particularly limited.

Furthermore, a plurality of bases may be used in combination.

The base is not particularly limited, and examples thereof include oil-based bases and aqueous bases. Among the bases, the oil-based bases are preferable. When the base is an oil-based base, a preparation having an S/O (Solid in Oil) structure can be formed by dispersing the core-shell structure in the oil-based base. The preparation having an S/O (Solid in Oil) structure can be produced, for example, by a method including a step of drying a W/O emulsion containing an active ingredient in an aqueous phase, as described below.

Examples of the oil-based base include vegetable oils, animal oils, neutral lipids, synthetic fats and oils, sterol derivatives, waxes, hydrocarbons, monoalcohol carboxylic acid esters, oxyacid esters, polyhydric alcohol fatty acid esters, silicones, higher alcohols, higher fatty acids, and fluorine-based oils. Examples of the aqueous base include water and (polyhydric) alcohols.

The vegetable oil is not particularly limited, and examples thereof include soy oil, sesame oil, olive oil, coconut oil, palm oil, rice oil, cottonseed oil, sunflower oil, rice bran oil, cacao butter, corn oil, safflower oil, castor oil, and rapeseed oil.

The animal oil is not particularly limited, and examples thereof include mink oil, turtle oil, fish oil, beef tallow oil, horse oil, lard, and shark squalane.

The neutral lipid is not particularly limited, and examples thereof include triolein, trilinolein, trimyristin, tristearin, and triarachidonin.

The synthetic fats and oils are not particularly limited, and examples thereof include phospholipids and azone.

The sterol derivative is not particularly limited, and examples thereof include dihydrocholesterol, lanosterol, dihydrolanosterol, phytosterol, cholic acid, and cholesteryl linoleate.

Examples of the wax include candelilla wax, carnauba wax, rice wax, Japanese wax, beeswax, montan wax, ozokerite, ceresin, paraffin wax, microcrystallin wax, petrolatum, Fischer-Tropsch wax, polyethylene wax, and ethylene/propylene copolymers.

Examples of the hydrocarbon include liquid paraffin (mineral oil), heavy liquid isoparaffin, light liquid isoparaffin, $\alpha$-olefin oligomer, polyisobutene, hydrogenated polyisobutene, polybutene, squalane, olive-derived squalane, squalene, vaseline, and solid paraffin.

Examples of the monoalcohol carboxylic acid ester include octyldodecyl myristate, hexyldecyl myristate, octyldodecyl isostearate, cetyl palmitate, octyldodecyl palmitate, cetyl octanoate, hexyldecyl octanoate, isotridecyl isononanoate, isononyl isononanoate, octyl isononanoate, isotridecyl isononanoate, isodecyl neopentanoate, isotridecyl neopentanoate, isostearyl neopentanoate, octyldodecyl neodecanoate, oleyl oleate, octyldodecyl oleate, octyldodecyl ricinoleate, octyldodecyl lanolate, hexyldecyl dimethyloctanoate, octyldodecyl erucate, hydrogenated castor oil isostearate, ethyl oleate, ethyl avocadate, isopropyl myristate, isopropyl palmitate, octyl palmitate, isopropyl isostearate, isopropyl lanolate, diethyl sebacate, diisopropyl sebacate, dioctyl sebacate, diisopropyl adipate, dibutyloctyl sebacate, diisobutyl adipate, dioctyl succinate, and triethyl citrate.

Examples of the oxyacid ester include cetyl lactate, diisostearyl malate, and hydrogenated castor oil monoisostearate.

Examples of polyhydric alcohol fatty acid ester include glyceryl trioctanoate, glyceryl trioleate, glyceryl triisostearate, glyceryl diisostearate, caprylic/capric triglyceride, caprylic/capric/myristic/stearic triglyceride, hydrogenated rosin triglyceride (hydrogenated ester gum), rosin triglyceride (ester gum), glyceryl behenate/eicosadioate, trimethylolpropane trioctanoate, trimethylolpropane triisostearate, neopentyl glycol dioctanoate, neopentyl glycol dicaprate, 2-butyl-2-ethyl-1,3-propanediol dioctanoate, propylene glycol dioleate, pentaerythrityl tetraoctanoate, hydrogenated rosin pentaerythrityl, ditrimethylolpropane triethylhexanoate, ditrimethylolpropane isostearate/sebacate, pentaerythrityl triethylhexanoate, dipentaerythrityl hydroxystearate/stearate/rosinate, diglyceryl diisostearate, polyglyceryl tetraisostearate, polyglyceryl-10 nonaisostearate, polyglyceryl-8 decaerucate/decaisostearate/decaricinoleate, diglyceryl sebacate/isopalmitate, glycol distearate (ethylene glycol distearate), 3-methyl-1,5-pentanediol dineopentanoate, and 2,4-diethyl-1,5-pentanediol dineopentanoate.

Examples of the silicone include dimethicone (dimethylpolysiloxane), highly polymerized dimethicone (highly polymerized dimethylpolysiloxane), cyclomethicone (cyclic dimethylsiloxane, decamethylcyclopentasiloxane), phenyl trimethicone, diphenyl dimethicone, phenyl dimethicone, stearoxypropyl dimethylamine, (aminoethylaminopropyl methicone/dimethicone) copolymers, dimethiconol, dimethiconol crosspolymer, silicone resins, silicone rubber, amino-modified silicones such as aminopropyl dimethicone and amodimethicone, cation-modified silicones, polyether-modified silicones such as dimethicone copolyol, polyglycerin-modified silicones, sugar-modified silicones, carboxylic acid-modified silicones, phosphoric acid-modified silicones, sulfuric acid-modified silicones, alkyl-modified silicones, fatty acid-modified silicones, alkyl ether-modified silicones, amino acid-modified silicones, peptide-modified silicones, fluorine-modified silicones, cation-modified or polyether-modified silicones, amino-modified or polyether-modified silicones, alkyl-modified or polyether-modified silicones, and polysiloxane/oxyalkylene copolymers.

Examples of the higher alcohol include cetanol, myristyl alcohol, oleyl alcohol, lauryl alcohol, cetostearyl alcohol, stearyl alcohol, arachyl alcohol, behenyl alcohol, jojoba alcohol, chimyl alcohol, selachyl alcohol, batyl alcohol, hexyldecanol, isostearyl alcohol, 2-octyldodecanol, and dimerdiol.

Examples of the higher fatty acid include lauric acid, myristic acid, palmitic acid, stearic acid, isostearic acid, behenic acid, undecylenic acid, 12-hydroxystearic acid, palmitoleic acid, oleic acid, linoleic acid, linolenic acid, erucic acid, docosahexaenoic acid, eicosapentaenoic acid, isohexadecanoic acid, anteiso heneicosanoic acid, long-chain branched fatty acids, dimer acid, and hydrogenated dimer acid.

Examples of the fluorine-based oil include perfluorodecane, perfluorooctane, and perfluoropolyether.

Examples of the (polyhydric) alcohol include ethanol, isopropanol, glycerin, propylene glycol, 1,3-butylene glycol, and polyethylene glycol.

Another base is not particularly limited, and examples thereof include bases used in a tape preparation such as a plaster (reservoir type tape preparation, matrix type tape preparation, or the like), an adhesive preparation such as a poultice, a patch, or a microneedle, an ointment, an external liquid preparation (such as a liniment or a lotion), a spray (such as an external aerosol or a pump spray preparation), a cream, a gel, an eye drop, an eye ointment, a nasal drop, a suppository, a semisolid preparation for rectal, an enema agent, an oral agent, or an injection.

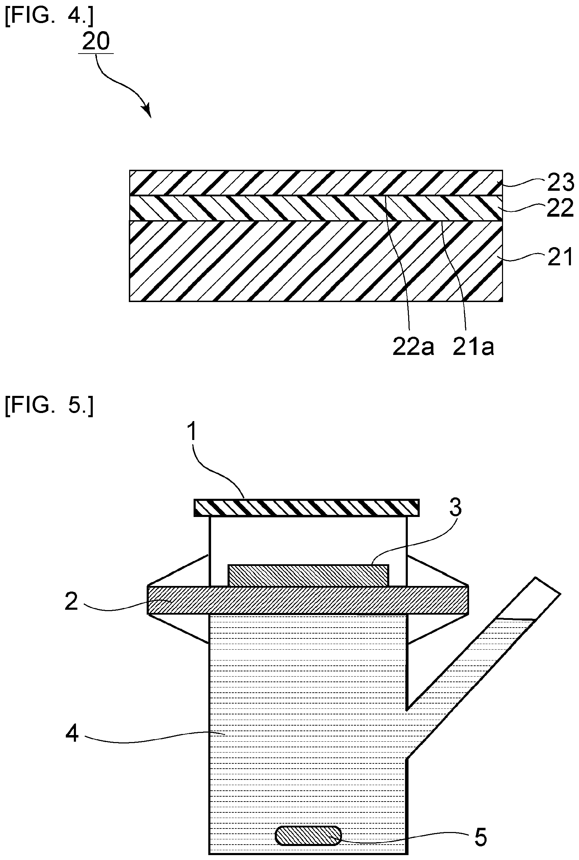

Hereinafter, an example of the tape preparation according to the present invention will be described with reference to Fig. 4.

Fig. 4 is a schematic sectional view showing a tape preparation according to one embodiment of the present invention.

As shown in Fig. 4, a tape preparation 20 includes a substrate layer 21 and an adhesive layer 22. The substrate layer 21 has a surface 21a laminated with the adhesive layer 22. The adhesive layer 22 has a surface 22a laminated with a liner 23.

As in the present embodiment, only the surface 21a on one side of the substrate layer 21 may be laminated with the adhesive layer 22, while both sides may be laminated. The adhesive layer 22 of the tape preparation 20 includes the core-shell structure according to the present invention. However, in a reservoir type tape preparation and the like, the core-shell structure does not need to be included in the adhesive layer 22, and may be included in, for example, a storage phase.

The substrate layer 21 is not particularly limited as long as it supports the adhesive layer 22, and examples of the substrate layer 21 include resin films, fibers, and non-woven fabrics. Examples of the resin film include polyester films and polyolefin films. The resin film is preferably a polyester film. Examples of the polyester include polyethylene terephthalate and polybutylene phthalate, and polyethylene terephthalate is preferable.

The adhesive included in the adhesive layer 22 is not particularly limited, and examples of the adhesive include rubber-based adhesives, acryl-based adhesives, and silicone-based adhesives. The adhesive included in the adhesive layer 22 is preferably a rubber-based adhesive or an acryl-based adhesive, and more preferably an acryl-based adhesive.

The liner 23 is not particularly limited as long as it protects the adhesive layer 22 until the tape preparation 20 is applied to the skin, and as long as the liner is coated with silicone or the like to be easily peeled off. Examples of the liner 23 include polyethylene terephthalate and polypropylene that are coated with silicone. The liner 23 does not need to be provided. In the formation of the adhesive layer 22, the adhesive may be applied to the substrate 21 side or the liner 23 side.

The tape preparation 20 of the present embodiment has the adhesive layer 22 including the core-shell structure according to the present invention. Therefore, both improvement in the transdermal absorption of the active ingredient and reduction in the irritation can be achieved at a high level. Furthermore, the tape preparation 20 can achieve both the adhesive strength and the holding strength at a high level since the core-shell structure according to the present invention included in the adhesive layer 22 contains both the first surfactant having a melting point of less than 35°C and the second surfactant having a melting point of 35°C or more.

[Production method]

The method of producing the core-shell structure according to the present invention is not particularly limited, and the core-shell structure can be produced, for example, by a method including a step of drying a W/O emulsion containing an active ingredient in an aqueous phase.

The W/O emulsion is not particularly limited as long as it is a so-called water-in-oil emulsion, specifically, an emulsion in which droplets of an aqueous solvent are dispersed in an oil solvent.

The W/O emulsion containing an active ingredient in an aqueous phase can be obtained, for example, by mixing an aqueous solvent such as water or an aqueous buffer solution containing an active ingredient with an oil solvent such as cyclohexane, hexane, or toluene containing a surfactant. The aqueous solvent containing an active ingredient may contain, in addition to the active ingredient, an additive ingredient such as a stabilizing agent, an absorption promoting agent, or an irritation reducing agent if necessary. The oil solvent containing a surfactant may contain, in addition to the surfactant, an additive ingredient such as an irritation reducing agent, an analgesic, an absorption promoting agent, or a stabilizing agent if necessary. The method of mixing is not particularly limited as long as a W/O emulsion can be formed by the method, and is, for example, stirring with a homogenizer or the like.

The condition for stirring with a homogenizer is, for example, about 5,000 rpm to 50,000 rpm, and preferably about 10,000 rpm to 30,000 rpm.

In the W/O emulsion, the mass ratio of the active ingredient to the first surfactant and the second surfactant (the active ingredient : the total of the first surfactant and the second surfactant) is preferably in the range of 1 : 0.5 to 1 : 100, and more preferably in the range of 1 : 5 to 1 : 100. The mass ratio is still more preferably in the range of 1 : 0.5 to 1 : 50, and particularly preferably in the range of 1 : 5 to 1 : 50. Furthermore, the mass ratio is still more preferably in the range of 1 : 0.5 to 1 : 30, and particularly preferably in the range of 1 : 5 to 1 : 30. The mass ratio may be 1 : 0.5 to 1 : 2.

The method of drying the W/O emulsion containing an active ingredient in an aqueous phase is not particularly limited as long as the solvents (the aqueous solvent and the oil solvent) in the emulsion can be removed by the method. Examples of the method of drying the W/O emulsion include freeze-drying and reduced-pressure drying, and the freeze-drying is preferable.

From the viewpoint of further reducing the number average particle size of the obtained core-shell structure, the method preferably further includes a step of heat-treating the W/O emulsion or the dried product of the W/O emulsion. The heat treatment temperature is, for example, 30°C to 60°C, preferably 35°C to 50°C, and more preferably 35°C to 45°C.

The heat treatment time is appropriately adjusted according to the heat treatment temperature, and is, for example, 1 day to 30 days, preferably 2 days to 15 days, and more preferably 3 to 7 days.

Examples of another method of further reducing the number average particle size of the obtained core-shell structure include filtration with a filter or the like and centrifugal separation that are performed after dispersing the W/O emulsion or the dried product of the W/O emulsion in a solvent or the like as necessary. In the case of filtration with a filter, the filter pore size is, for example, 1 $\mu$m or less, preferably 0.2 $\mu$m or less, and more preferably 0.1 $\mu$m or less.

The core-shell structure according to the present invention may be used as it is, or may be dispersed in the above-described base or the like and used.

A preparation can be produced using the core-shell structure according to the present invention by, for example, a solution coating method. In the solution coating method, the core-shell structure according to the present invention, the base, and, in addition, a desired additive ingredient are added to a solvent at a predetermined ratio. Then, the resulting mixture is stirred to prepare a uniform solution. Examples of the additive ingredient include absorption promoting agents, thickeners, and gelling agents. Examples of the solvent include hexane, toluene, and ethyl acetate. The solution preferably has a solid content concentration of 10% by mass to 80% by mass, and more preferably 20% by mass to 60% by mass.

Next, the solution containing the ingredients is uniformly applied, using a coating machine such as a knife coater, a comma coater, or a reverse coater, to a release liner such as a silicone-treated polyester film. After the application, the resulting product is dried to complete a drug-containing layer, and the drug-containing layer is laminated with a supporting member to obtain a preparation. A preparation may be produced, according to the kind of the supporting member, by forming a drug-containing layer on the supporting member and then laminating the surface of the drug-containing layer with a release liner.

In another method, for example, first, an additive ingredient such as a base, an absorption promoting agent, a stabilizing agent, a thickener, or a gelling agent is added to the core-shell structure according to the present invention as necessary and the resulting mixture is mixed. After the mixing, a member depending on the use is laminated or impregnated with the mixture to hold the mixture. Examples of the member include natural woven fabric members such as gauze and absorbent cotton, synthetic fiber woven fabric members such as polyester and polyethylene, woven fabrics and non-woven fabrics processed by appropriately combining the above-described members, and permeable films. The resulting product can be further covered with an adhesive cover material or the like and used.

The preparation thus obtained is appropriately cut into a shape such as an ellipse, a circle, a square, or a rectangle depending on the use. If necessary, an adhesive layer or the like may be provided in the periphery.

Next, the present invention will be clarified by giving specific Examples and Comparative Examples of the present invention. Note that the present invention is not limited to Examples shown below.

(Example 1)

Preparation of core-shell structure

In 40 g of pure water, 0.2 g of vardenafil hydrochloride hydrate (manufactured by Atomax Chemicals Co., Ltd., octanol/water partition coefficient: 0.0, molecular weight: 579 g/mol) as the active ingredient was dissolved, and to the resulting solution, a solution was added that was prepared by dissolving 0.075 g of glyceryl monocaprylate (manufactured by Taiyo Kagaku Co., Ltd., product name: "Sunsoft No. 700P-2-C", HLB value: 10.9, carbon number of saturated hydrocarbon group: 7, melting point: 27°C) as the first surfactant and 0.025 g of glyceryl monocaprate (manufactured by Taiyo Kagaku Co., Ltd., product name: "Sunsoft No. 760-C", HLB value: 9.7, carbon number of saturated hydrocarbon group: 9, melting point: 53°C) as the second surfactant in 80 g of cyclohexane, and the resulting mixture was stirred with a homogenizer (10,000 rpm, 2 minutes). Then, the mixture was freeze-dried for two days to obtain a core-shell structure.

The melting point was determined from the endothermic peak in measurement by differential scanning calorimetry (DSC) described below. As the measuring device, DSC6220 (manufactured by Hitachi High-Tech Science Corporation) was used. In Examples and Comparative Examples described below, the melting point was determined in the same manner as in Example 1.

[Measurement conditions]

Sample amount: 10 mg
Temperature condition: holding at -150°C for 20 minutes and heating to 75°C at a rate of 6°C/min after the holding.
Measurement atmosphere: under nitrogen atmosphere

Preparation of ointment

With 60 parts by weight of the obtained core-shell structure, 40 parts by weight of an ointment base, Plastibase (manufactured by Taisho Pharmaceutical Co., Ltd.) was blended, mixed, and dispersed to prepare an ointment.

(Example 2)

A core-shell structure and an ointment were prepared in the same manner as in Example 1 except that the amount of glyceryl monocaprylate blended as the first surfactant was 0.05 g and the amount of glyceryl monocaprate blended as the second surfactant was 0.05 g.

(Example 3)

A core-shell structure and an ointment were prepared in the same manner as in Example 1 except that the amount of glyceryl monocaprylate blended as the first surfactant was 0.025 g and the amount of glyceryl monocaprate blended as the second surfactant was 0.075 g.

(Example 4)

Preparation of core-shell structure

In 40 g of pure water, 0.2 g of vardenafil hydrochloride hydrate (manufactured by Atomax Chemicals Co., Ltd.,

octanol/water partition coefficient: 0.0, molecular weight: 579 g/mol) as the active ingredient was dissolved, and to the resulting solution, a solution was added that was prepared by dissolving 0.075 g of glyceryl monocaprylate (manufactured by Taiyo Kagaku Co., Ltd., product name: "Sunsoft No. 700P-2-C", HLB value: 10.9, carbon number of saturated hydrocarbon group: 7, melting point: 27°C) as the first surfactant and 0.025 g of glyceryl monocaprate (manufactured by Taiyo Kagaku Co., Ltd., product name: "Sunsoft No. 760-C", HLB value: 9.7, carbon number of saturated hydrocarbon group: 9, melting point: 53°C) as the second surfactant in 80 g of cyclohexane, and the resulting mixture was stirred with a homogenizer (10,000 rpm, 2 minutes). Then, the mixture was freeze-dried for two days to obtain a core-shell structure.

Preparation of tape preparation

With 40 parts by weight of the obtained core-shell structure, 60 parts by weight of acrylic adhesive (manufactured by CosMED Pharmaceutical Co. Ltd., product name: "MAS-683") was blended, and toluene was added so that the solid content concentration was 40% by weight. Then, the resulting mixture was mixed until uniform to prepare an adhesive layer solution.

Next, a release sheet was prepared that had been release-treated by applying silicone to one surface of a release substrate including a polyethylene terephthalate film having a thickness of 38 $\mu$m. The adhesive layer solution was applied to the release-treated surface of the release sheet, and the resulting product was dried at 90°C for 20 minutes to prepare a laminate having a 100 $\mu$m-thick adhesive layer formed on the release-treated surface of the release sheet. Then, a supporting member including a polyethylene terephthalate film having a thickness of 38 $\mu$m was prepared. The supporting member and the laminate were stacked so that one surface of the supporting member faced the adhesive layer of the laminate, and the adhesive layer of the laminate was transferred to the supporting member to integrate the stacked supporting member and the laminate. As a result, a tape preparation was prepared.

(Example 5)

A core-shell structure and a tape preparation were prepared in the same manner as in Example 4 except that the amount of glyceryl monocaprylate blended as the first surfactant was 0.05 g and the amount of glyceryl monocaprate blended as the second surfactant was 0.05 g.

(Example 6)

A core-shell structure and a tape preparation were prepared in the same manner as in Example 4 except that the amount of glyceryl monocaprylate blended as the first surfactant was 0.025 g and the amount of glyceryl monocaprate blended as the second surfactant was 0.075 g.

(Comparative Example 1)

A core-shell structure and an ointment were prepared in the same manner as in Example 1 except that as the surfactant, only glyceryl monocaprylate as the first surfactant was used in an amount of 0.1 g without using the second surfactant.

(Comparative Example 2)

A core-shell structure and an ointment were prepared in the same manner as in Example 1 except that as the surfactant, only glyceryl monocaprate as the second surfactant was used in an amount of 0.1 g without using the first surfactant.

(Comparative Example 3)

A core-shell structure and a tape preparation were prepared in the same manner as in Example 4 except that as the surfactant, only glyceryl monocaprylate as the first surfactant was used in an amount of 0.1 g without using the second surfactant.

(Comparative Example 4)

A core-shell structure and a tape preparation were prepared in the same manner as in Example 4 except that as the surfactant, only glyceryl monocaprate as the second surfactant was used in an amount of 0.1 g without using the first surfactant.

(Evaluation)

With respect to the ointment obtained in Examples 1 to 3 and Comparative Examples 1 and 2, the hairless rat skin permeability and the rabbit skin primary irritation were evaluated by the following tests.

Hairless rat skin permeability test

A hairless rat skin (manufactured by Japan SLC, Inc., extracted from 8 weeks old HWY/Slc) was set in a cell for a skin permeation test for a drug (Fig. 5). To the upper part of this device, 0.8 g (1.33 cm$^2$) of the ointment obtained in Examples 1 to 3 and Comparative Examples 1 and 2 was applied. A distilled water solution containing $5 \times 10^{-4}$ M of NaH$_2$PO$_4$, $2 \times 10^{-4}$ M of Na$_2$HPO$_4$, $1.5 \times 10^{-4}$ M of NaCl, and 10 ppm of gentamicin sulfate (manufactured by Wako Pure Chemical Industries, Ltd., G1658) was adjusted to have a pH of 7.2 with NaOH to prepare a buffer solution, and the buffer solution was put into a receptor layer in the lower part. The device was installed in a thermostatic chamber kept at 32°C from the start of the test. After a predetermined time from the start of the test, 1 ml of the solution in the chamber was taken out from the receptor layer in the lower part, and immediately after that, 1 ml of a solution having the same composition was put into the receptor layer. To the receptor solution sample taken out, methanol was added to extract an eluted lipid or the like, and the resulting product was centrifuged. After the centrifugation, the concentration of the active ingredient in the supernatant was quantified by high performance liquid chromatography (HPLC). From the quantified amount of the active ingredient, the cumulative skin permeation amount for 24 hours was calculated.
In Examples 1 to 3 and Comparative Examples 1 and 2, the evaluation in the skin permeability test was performed in accordance with the following evaluation criteria.

[Evaluation criteria]

A ... Permeation amount after 24 hours is 10,000 μg/cm$^2$ or more
B ... Permeation amount after 24 hours is 1,000 μg/cm$^2$ or more and less than 10,000 μg/cm$^2$
C ... Permeation amount after 24 hours is less than 1,000 μg/cm$^2$

Rabbit skin primary irritation test

The dorsal skin of a rabbit was shaved with an electric clipper (with an electric shaver if necessary). On the dorsal healthy skin, two points in each of right and left sides of the dorsal midline, that is, four points in total were set to be an administration site. The ointment obtained in Examples 1 to 3 and Comparative Examples 1 and 2 was taken out with a spatula and spread evenly on a piece of lint having a size of 2 cm × 2 cm, and the piece of lint was attached to the administration site. The piece of lint was fixed by covering with a non-woven adhesive bandage (manufactured by Nichiban Co., Ltd., MESHPORE, No. 50). Then, the administration sites were altogether wrapped with gauze and enclosed by covering with an adhesive cloth elastic bandage (manufactured by Nichiban Co., Ltd., ELASTOPORE, No. 100). The enclosure was released 24 hours after the start of administration, and the administered samples were removed.
The skin reaction was visually observed with the naked eye 24 hours after the administration (30 minutes after the release of the enclosure and the removal of the administered samples). Then, furthermore, the skin reaction was visually observed with the naked eye 48 hours and 72 hours after the administration (30 minutes after the release of the enclosure and the removal of the administered samples) in the same manner. The skin reaction was evaluated in accordance with the Draize criteria shown in Table 2 below.

[Table 2]

| Degree of skin reaction | Score |
|---|---|
| Erythema/eschar formation | |
| No erythema | 0 |
| Very slight erythema (in barely detectable degree) | 1 |
| Apparent erythema | 2 |
| Moderate to severe erythema | 3 |

(continued)

| Degree of skin reaction | Score |
|---|---|
| Severe erythema with deep red color and slight eschar formation (with deep injury) | 4 |
| Edema formation | |
| No edema | 0 |
| Very slight edema (in barely detectable degree) | 1 |
| Apparent edema (well defined from surrounding area) | 2 |
| Moderate edema (raised by approximately 1 mm) | 3 |
| Severe edema (raised by 1 mm or more and extending to surrounding area) | 4 |

Specifically, for each administered sample at each time of observation, the individual score of the skin reaction at the administration site of each rabbit (the total of scores of erythema/eschar formation and edema formation) was calculated. Then, the primary irritation index (P.I.I.) was calculated from the individual scores at 24 hours and 72 hours after the administration (the score at 48 hours after the administration was not added). Specifically, the primary irritation index was calculated using Formulae (1) and (2) described below.

Average score of each administration site = (total of individual scores at 24 hours and 72 hours after administration)/2 ... Formula (1)

Primary irritation index (P.I.I.) = (total of average scores of administration sites)/(3 (rabbits)) ... Formula (2)

From the obtained primary irritation index (P.I.I.), the degree of irritation of each administered sample was classified in accordance with the classification table in Table 3 below.

[Table 3]

| Primary irritation index (P.I.I.) | Safety classification |
|---|---|
| **0** | No irritation |
| **O<P. I. I. ≦2** | Mild irritation |
| **2<P. I. I. ≦5** | Moderate irritation |
| **5<P. I. I.** | Severe irritation |

In Examples 1 to 3 and Comparative Examples 1 and 2, the evaluation in the irritation test was performed in accordance with the following evaluation criteria.

[Evaluation criteria]

A ... P.I.I. is 1.5 or less
B ... P.I.I. is more than 1.5 and 2.5 or less
C ... P.I.I. is more than 2.5

With respect to the tape preparation obtained in Examples 4 to 6 and Comparative Examples 3 and 4, the hairless rat skin permeability and the rabbit skin primary irritation were evaluated by the following tests. In addition, the tape peeling force and the tape holding force were evaluated by the following tests.

Hairless rat skin permeability test

A hairless rat skin (manufactured by Japan SLC, Inc., extracted from 8 weeks old HWY/Slc) was set in a cell for a skin permeation test for a drug (Fig. 5). To the upper part of this device, 1.33 cm$^2$ of the tape preparation obtained in Examples 4 to 6 and Comparative Examples 3 and 4 was applied. A distilled water solution containing $5 \times 10^{-4}$ M of NaH$_2$PO$_4$, $2 \times 10^{-4}$ M of Na$_2$HPO$_4$, $1.5 \times 10^{-4}$ M of NaCl, and 10 ppm of gentamicin sulfate (manufactured by Wako Pure Chemical Industries, Ltd., G1658) was adjusted to have a pH of 7.2 with NaOH to prepare a buffer solution, and the buffer solution was put into a receptor layer in the lower part. The device was installed in a thermostatic chamber kept at 32°C from the start of the test. After a predetermined time from the start of the test, 1 ml of the solution in the chamber was taken out from the receptor layer in the lower part, and immediately after that, 1 ml of a solution having the same composition was put into the receptor layer. To the receptor solution sample taken out, methanol was added to extract an eluted lipid or the like, and the resulting product was centrifuged. After the centrifugation, the concentration of the active ingredient in the supernatant was quantified by high performance liquid chromatography (HPLC). From the quantified amount of the active ingredient, the cumulative skin permeation amount for 24 hours was calculated.
In Examples 4 to 6 and Comparative Examples 3 and 4, the evaluation in the skin permeability test was performed in accordance with the following evaluation criteria.

[Evaluation criteria]

A ... Permeation amount after 24 hours is 15 $\mu$g/cm$^2$ or more
B ... Permeation amount after 24 hours is 3 $\mu$g/cm$^2$ or more and less than 15 $\mu$g/cm$^2$
C ... Permeation amount after 24 hours is less than 3 $\mu$g/cm$^2$

Rabbit skin primary irritation test

The dorsal skin of a rabbit was shaved with an electric clipper (with an electric shaver if necessary). On the dorsal healthy skin, two points in each of right and left sides of the dorsal midline, that is, four points in total were set to be an administration site. The tape preparation obtained in Examples 4 to 6 and Comparative Examples 3 and 4 was cut into a size of 2 cm $\times$ 2 cm, then the release sheet was peeled off, and the tape preparation was attached to the administration site. The tape preparation was fixed by covering with a non-woven adhesive bandage (manufactured by Nichiban Co., Ltd., MESHPORE, No. 50). Then, the administration sites were altogether wrapped with gauze and enclosed by covering with an adhesive cloth elastic bandage (manufactured by Nichiban Co., Ltd., ELASTOPORE, No. 100). The enclosure was released 24 hours after the start of administration, and the administered samples were removed.
The skin reaction was visually observed with the naked eye 24 hours after the administration (30 minutes after the release of the enclosure and the removal of the administered samples). Then, furthermore, the skin reaction was visually observed with the naked eye 48 hours and 72 hours after the administration (30 minutes after the release of the enclosure and the removal of the administered samples) in the same manner. The skin reaction was evaluated in accordance with the Draize criteria shown in Table 2 described above.
Specifically, for each administered sample at each time of observation, the individual score of the skin reaction at the administration site of each rabbit (the total of scores of erythema/eschar formation and edema formation) was calculated. Then, the primary irritation index (P.I.I.) was calculated from the individual scores at 24 hours and 72 hours after the administration (the score at 48 hours after the administration was not added). Specifically, the primary irritation index was calculated using Formulae (1) and (2) described below.

Average score of each administration site = (total of individual scores at 24 hours and 72 hours after administration)/2 ... Formula (1)

$$\text{Primary irritation index (P.I.I.)} = \text{(total of average scores of administration sites)}/(3 \text{ (rabbits))} \ldots \text{Formula (2)}$$

From the obtained primary irritation index (P.I.I.), the degree of irritation of each administered sample was classified in accordance with the classification table in Table 3 described above.

In Examples 4 to 6 and Comparative Examples 3 and 4, the evaluation in the irritation test was performed in accordance with the following evaluation criteria.

[Evaluation criteria]

A ... P.I.I. is 1.5 or less
B ... P.I.I. is more than 1.5 and 2.5 or less
C ... P.I.I. is more than 2.5

Tape peeling force test

An adhesive preparation having a width of 24 mm and a length of 100 mm was adhered to a SUS plate to prepare a test piece in accordance with JIS Z 0237: 2009 and peeled in the 90° direction. The strength at the time of peeling off was regarded as the peeling force. The peeling force was determined by measurement with a tensile tester. As the tensile tester, the product number "SVZ-50NB-1R1" manufactured by IMADA SEISAKUSHO CO., LTD. was used. The peeling rate was set to 300 mm/min.

In Examples 4 to 6 and Comparative Examples 3 and 4, the evaluation in the tape peeling force test was performed in accordance with the following evaluation criteria.

[Evaluation criteria]

A ... Tape peeling force is 500 mN/mm or more
B ... Tape peeling force is 100 mN/mm or more and less than 500 mN/mm
C ... Tape peeling force is less than 100 mN/mm

Tape holding force (holding time) test

An adhesive preparation having a width of 12 mm and a length of 12 mm was adhered to a SUS plate in accordance with JIS Z 0237: 2009. A 1.0 kg weight was attached to the end of the adhesive preparation, and from the time of the attachment, the elapsed time (holding time) was measured until the adhesive preparation was completely peeled off from the SUS plate. As the holding force tester, the product number "BE-502" manufactured by TESTER SANGYO CO., LTD. was used.

In Examples 4 to 6 and Comparative Examples 3 and 4, the evaluation in the tape holding force test was performed in accordance with the following evaluation criteria.

[Evaluation criteria]

A ... Tape holding force is 500s or more
B ... Tape holding force is 100s or more and less than 500 s
C ... Tape holding force is less than 100s

Table 4 described below shows the results of the hairless rat skin permeability test and the rabbit skin primary irritation test of the ointment obtained in Examples 1 to 3 and Comparative Examples 1 and 2. In addition, Table 4 described below shows the results of the hairless rat skin permeability test, the rabbit skin primary irritation test, the tape peeling force test, and the tape holding force test of the tape preparation obtained in Examples 4 to 6 and Comparative Examples 3 and 4.

[Table 4]

| | First surfactant (melting point: less than 35°C) | | | | Second surfactant (melting point: 35°C or more) | | | | Mass ratio of first surfactant to second surfactant | | Form of preparation | Irritation (P.I.I.) | | Permeation amount after 24 hours ($\mu g/cm^2$) | | Tape peeling force (mN/mm) | | Tape holding force (s) | |
|---|---|---|---|---|---|---|---|---|---|---|---|---|---|---|---|---|---|---|---|
| | Name | Hydrophilie moiety structure | Carbon number in hydrocarbon chain | Melting point (°C) | Name | Hydrophilic moiety structure | Carbon number in hydrocarbon chain | Melting point (°C) | First surfactant | Second surfactant | | | | | | | | | |
| Example 1 | Glyceryl monocapr-ylate | Glycerin | 7 | 27 | Glyceryl monocaprate | Glycerin | 9 | 53 | 75 | 25 | Ointment | 1.0 | A | 4737 | B | - | | - | |
| Example 2 | Glyceryl monocapr-ylate | Glycerin | 7 | 27 | Glyceryl monocaprate | Glycerin | 9 | 53 | 50 | 50 | Ointment | 1.0 | A | 11333 | A | - | | - | |
| Example 3 | Glyceryl monocapr-ylate | Glycerin | 7 | 27 | Glyceryl monocaprate | Glycerin | 9 | 53 | 25 | 75 | Ointment | 1.0 | A | 17053 | A | - | | - | |
| Example 4 | Glyceryl monocapr-ylate | Glycerin | 7 | 27 | Glyceryl monocaprate | Glycerin | 9 | 53 | 75 | 25 | Tape preparation | 1.0 | A | 4.7 | B | 557.4 | A | 147 | B |
| Example 5 | Glyceryl monocapr-ylate | Glycerin | 7 | 27 | Glyceryl monocaprate | Glycerin | 9 | 53 | 50 | 50 | Tape preparation | 1.0 | A | 8.5 | B | 286.7 | B | 214 | B |
| Example 6 | Glyceryl monocapr-ylate | Glycerin | 7 | 27 | Glyceryl monocaprate | Glycerin | 9 | 53 | 25 | 75 | Tape preparation | 1.0 | A | 19.3 | A | 360.2 | B | 654 | A |
| Comparative Example 1 | Glyceryl monocapr-ylate | Glycerin | 7 | 27 | - | - | - | - | 100 | 0 | Ointment | 1.0 | A | 709 | C | - | | - | |
| Comparative Example 2 | - | - | - | - | Glyceryl monocaprate | Glycerin | 9 | 53 | 0 | 100 | Ointment | 3.8 | C | 15141 | A | - | | - | |

EP 3 950 071 A1

24

| | First surfactant (melting point: less than 35°C) | | | | Second surfactant (melting point: 35°C or more) | | | | Mass ratio of first surfactant to second surfactant | | Form of preparation | Irritation (P.I.I.) | | Permeation amount after 24 hours (μg/cm$^2$) | | Tape peeling force (mN/mm) | | Tape holding force (s) | |
|---|---|---|---|---|---|---|---|---|---|---|---|---|---|---|---|---|---|---|---|
| | Name | Hydrophilie moiety structure | Carbon number in hydrocarbon chain | Melting point (°C) | Name | Hydrophilic moiety structure | Carbon number in hydrocarbon chain | Melting point (°C) | First surfactant | Second surfactant | | | | | | | | | |
| Comparative Example 3 | Glyceryl monocapryl ate | Glycerin | 7 | 27 | - | - | - | - | 100 | 0 | Tape preparation | 1.0 | A | 2.6 | C | 333.4 | B | 33 | C |
| Comparative Example 4 | - | - | - | - | Glyceryl monocaprate | Glycerin | 9 | 53 | 0 | 100 | Tape preparation | 3.0 | C | 25.2 | A | 34.4 | C | 1127 | A |

As shown in Table 4, it can be confirmed that the ointment in Examples 1 to 3 and the tape preparation in Examples 4 to 6 can achieve both high transdermal absorption of the active ingredient and low irritation at a high level. Furthermore, it can be confirmed that the tape preparation in Examples 4 to 6 can achieve both the peeling force and the holding force at a high level.

(Example 7)

A core-shell structure and an ointment were prepared in the same manner as in Example 1 except that the amount of glyceryl monocaprylate blended as the first surfactant was 0.015 g and the amount of glyceryl monocaprate blended as the second surfactant was 0.085 g.

(Example 8)

A core-shell structure and an ointment were prepared in the same manner as in Example 1 except that the amount of glyceryl monocaprylate blended as the first surfactant was 0.055 g and the amount of glyceryl monocaprate blended as the second surfactant was 0.045 g.

(Example 9)

A core-shell structure and an ointment were prepared in the same manner as in Example 1 except that the amount of glyceryl monocaprylate blended as the first surfactant was 0.035 g and the amount of glyceryl monocaprate blended as the second surfactant was 0.065 g.

(Example 10)

A core-shell structure and an ointment were prepared in the same manner as in Example 9 except that glyceryl monopalmitate (manufactured by AccuStandard, product name: "Monopalmitin", HLB value: 7.2, carbon number of saturated hydrocarbon group: 15, melting point: 68°C) was used instead of glyceryl monocaprate as the second surfactant.

(Example 11)

A core-shell structure and an ointment were prepared in the same manner as in Example 9 except that glyceryl monomyristate (manufactured by NIPPON SURFACTANT INDUSTRIES CO., LTD., product name: "NIKKOL MGM", HLB value: 7.9, carbon number of saturated hydrocarbon group: 13, melting point: 46°C) was used instead of glyceryl monocaprate as the second surfactant.

(Example 12)

A core-shell structure and an ointment were prepared in the same manner as in Example 9 except that glyceryl monolaurate (manufactured by Taiyo Kagaku Co., Ltd., product name: "Sunsoft No. 750-C", HLB value: 8.7, carbon number of saturated hydrocarbon group: 11, melting point: 57°C) was used instead of glyceryl monocaprate as the second surfactant.

(Example 13)

A core-shell structure and an ointment were prepared in the same manner as in Example 9 except that sorbitan monopalmitate (manufactured by NIPPON SURFACTANT INDUSTRIES CO., LTD., product name: "NIKKOL SP-10V", HLB value: 9.5, carbon number of saturated hydrocarbon group: 15, melting point: 45°C) was used instead of glyceryl monocaprate as the second surfactant.

(Example 14)

A core-shell structure and an ointment were prepared in the same manner as in Example 9 except that sorbitan monomyristate (manufactured by NOF CORPORATION, product name: "NONION MP-30R", HLB value: 10.2, carbon number of saturated hydrocarbon group: 13, melting point: 36°C) was used instead of glyceryl monocaprate as the second surfactant.

(Example 15)

A core-shell structure and an ointment were prepared in the same manner as in Example 9 except that propylene glycol monocaprylate (manufactured by NIPPON SURFACTANT INDUSTRIES CO., LTD., product name: "NIKKOL SEFSOL-218", HLB value: 10.2, carbon number of saturated hydrocarbon group: 7, melting point: -50°C to 5°C) was used instead of glyceryl monocaprylate as the first surfactant.

With respect to the ointment obtained in Examples 7 to 15, the hairless rat skin permeability and the rabbit skin primary irritation were evaluated by the same tests as in Examples 1 to 3 and Comparative Examples 1 to 2. Table 5 described below shows the results.

[Table 5]

| | First surfactant (melting point: less than 35°C) | | | | Second surfactant (melting point: 35°C or more) | | | | Mass ratio of first surfactant to second surfactant | | Form of preparation | Irritation (P.I.I.) | | Permeation amount after 24 hours ($\mu$g/cm$^2$) | |
|---|---|---|---|---|---|---|---|---|---|---|---|---|---|---|---|
| | Name | Hydrophilic moiety structure | Carbon number in hydrocarbon chain | Melting point (°C) | Name | Hydrophilie moiety structure | Carbon number in hydrocarbon chain | Melting point (°C) | First surfactant | Second surfactant | | | | | |
| Example 7 | Glyceryl monocaprylate | Glycerin | 7 | 27 | Glyceryl mono caprate | Glycerin | 9 | 53 | 15 | 85 | Ointment | 1.8 | B | 16900 | A |
| Example 8 | Glyceryl monocaprylate | Glycerin | 7 | 27 | Glyceryl mono caprate | Glycerin | 9 | 53 | 55 | 45 | Ointment | 1.0 | A | 9033 | B |
| Example 9 | Glyceryl monocaprylate | Glycerin | 7 | 27 | Glyceryl mono caprate | Glycerin | 9 | 53 | 35 | 65 | Ointment | 1.0 | A | 17041 | A |
| Example 10 | Glyceryl monocaprylate | Glycerin | 7 | 27 | Glyceryl monopalmitate | Glycerin | 15 | 68 | 35 | 65 | Ointment | 1.0 | A | 5103 | B |
| Example 11 | Glyceryl monocaprylate | Glycerin | 7 | 27 | Glyceryl monomyristate | Glycerin | 13 | 46 | 35 | 65 | Ointment | 1.0 | A | 9096 | B |
| Example 12 | Glyceryl monocaprylate | Glycerin | 7 | 27 | Glyceryl monolaurate | Glycerin | 11 | 57 | 35 | 65 | Ointment | 1.0 | A | 14097 | A |
| Example 13 | Glyceryl monocaprylate | Glycerin | 7 | 27 | Sorbitan monopalmitate | Sorbitan | 15 | 45 | 35 | 65 | Ointment | 1.0 | A | 4012 | B |
| Example 14 | Glyceryl monocaprylate | Glycerin | 7 | 27 | Sorbitan monomyristate | Sorbitan | 13 | 36 | 35 | 65 | Ointment | 1.0 | A | 2016 | B |

| | First surfactant (melting point: less than 35°C) | | | | Second surfactant (melting point: 35°C or more) | | | | Mass ratio of first surfactant to second surfactant | | Form of preparation | Irritation (P.I.I.) | | Permeation amount after 24 hours ($\mu$g/cm$^2$) | |
| --- | --- | --- | --- | --- | --- | --- | --- | --- | --- | --- | --- | --- | --- | --- | --- |
| | Name | Hydrophilic moiety structure | Carbon number in hydrocarbon chain | Melting point (°C) | Name | Hydrophilic moiety structure | Carbon number in hydrocarbon chain | Melting point (°C) | First surfactant | Second surfactant | | | | | |
| Example 15 | Propylene glycol monocaprylate | Propylene glycol | 7 | -50~-5 | Glyceryl monocaprate | Glycerin | 9 | 53 | 35 | 65 | Ointment | 1.0 | A | 3159 | B |

EP 3 950 071 A1

As shown in Table 5, it can be confirmed that also the ointment obtained in Examples 7 to 15 can achieve both high transdermal absorption of the active ingredient and low irritation at a high level.

**EXPLANATION OF SYMBOLS**

| | |
|---|---|
| 1: | Parafilm |
| 2: | Skin |
| 3: | Preparation |
| 4: | Receptor solution (phosphate buffer solution having pH = 7.2) |
| 5: | Stirrer |
| 10: | Core-shell structure |
| 11: | Core portion |
| 12: | Shell portion |
| 20: | Tape preparation |
| 21: | Substrate layer |
| 21a,22a: | Surface |
| 22: | Adhesive layer |
| 23: | Liner |

**Claims**

1.  A core-shell structure comprising:

    a core portion containing an active ingredient,
    the core portion being solid; and
    a shell portion containing a first surfactant having a melting point of less than 35°C and a second surfactant having a melting point of 35°C or more,
    the first surfactant and the second surfactant each having a hydrophile lipophile balance (HLB) value of 4 to 14,
    the first surfactant and the second surfactant each containing at least one selected from the group consisting of sorbitan fatty acid esters, glycerin fatty acid esters, propylene glycol fatty acid esters, and fatty acid alkanolamides.

2.  The core-shell structure according to claim 1, wherein a mass ratio of the first surfactant to the second surfactant (the first surfactant : the second surfactant) is 10 : 90 to 80 : 20.

3.  The core-shell structure according to claim 1 or 2, wherein the first surfactant and the second surfactant each have a saturated hydrocarbon group having 5 to 15 carbon atoms or an unsaturated hydrocarbon group having 5 to 17 carbon atoms.

4.  The core-shell structure according to any one of claims 1 to 3, wherein each hydrocarbon group in the first surfactant has 5 or more and 8 or less carbon atoms.

5.  The core-shell structure according to any one of claims 1 to 4, wherein each hydrocarbon group in the second surfactant has 9 or more and 17 or less carbon atoms.

6.  The core-shell structure according to any one of claims 1 to 5, wherein the first surfactant and the second surfactant each include an ester bond or an amide bond formed between an alcohol and a fatty acid, and the alcohol has a molecular weight of 70 g/mol or more and 330 g/mol or less.

7.  The core-shell structure according to any one of claims 1 to 6, wherein the first surfactant and the second surfactant each contain at least one selected from the group consisting of sorbitan fatty acid esters, glycerin fatty acid esters, and propylene glycol fatty acid esters.

8.  The core-shell structure according to any one of claims 1 to 7, wherein a glycerin fatty acid ester contained in each of the first surfactant and the second surfactant is at least one selected from the group consisting of monoglycerin fatty acid esters, diglycerin fatty acid esters, and triglycerin fatty acid esters.

9. The core-shell structure according to any one of claims 1 to 8, wherein a mass ratio of the active ingredient to the first surfactant and the second surfactant (the active ingredient : a total of the first surfactant and the second surfactant) is 1 : 0.1 to 1 : 100.

10. A preparation comprising the core-shell structure according to any one of claims 1 to 9.

11. An external medicine comprising the core-shell structure according to any one of claims 1 to 9.

12. A tape preparation comprising the core-shell structure according to any one of claims 1 to 9.

13. A cosmetic comprising the core-shell structure according to any one of claims 1 to 9.

[FIG. 1.]

10

12

11

[FIG. 2.]

$$R - \overset{\overset{\displaystyle O}{\displaystyle \|}}{C} - O - R'$$

[FIG. 3.]

$$R - \overset{\overset{\displaystyle O}{\displaystyle \|}}{C} - N \overset{\displaystyle R'}{\underset{\displaystyle R''}{}}$$

[FIG. 4.]

[FIG. 5.]

<table>
<tr><td colspan="2" align="center">**INTERNATIONAL SEARCH REPORT**</td><td colspan="2">International application No.<br>PCT/JP2020/013934</td></tr>
</table>

A. CLASSIFICATION OF SUBJECT MATTER

Int.Cl. A61Q19/00(2006.01)i, A61K9/06(2006.01)i, A61K9/50(2006.01)i, A61K9/70(2006.01)i, A61L15/20(2006.01)i, A61L15/44(2006.01)i, A61L26/00(2006.01)i, A61K8/11(2006.01)i, A61K8/37(2006.01)i, A61K8/42(2006.01)i, A61K47/14(2006.01)i, A61K47/18(2006.01)i, A61K47/26(2006.01)i, A61K31/53(2006.01)i

FI: A61K9/50, A61K47/26, A61K47/14, A61K47/18, A61K9/70401, A61K31/53, A61K9/06, A61K8/11, A61K8/37, A61K8/42, A61Q19/00, A61L15/20100, A61L15/44100, A61L26/00

According to International Patent Classification (IPC) or to both national classification and IPC

B. FIELDS SEARCHED

Minimum documentation searched (classification system followed by classification symbols)

Int.Cl. A61Q19/00, A61K9/06, A61K9/50, A61K9/70, A61L15/20, A61L15/44, A61L26/00, A61K8/11, A61K8/37, A61K8/42, A61K47/14, A61K47/18, A61K47/26, A61K31/53

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

JSTPlus/JMEDPlus/JST7580(JDreamIII), CAplus/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| A | WO 2018/147333 A1 (SEKISUI CHEMICAL CO., LTD.) 16.08.2018 (2018-08-16), particularly, claims 1-13, examples 1-35, paragraphs [0070], [0071] | 1-13 |
| P, X | WO 2020/013241 A1 (SEKISUI CHEMICAL CO., LTD.) 16.01.2020 (2020-01-16), particularly, example 4, paragraphs [0036]-[0085], claims 1-10 | 1-13 |
| P, A | WO 2019/155992 A1 (SEKISUI CHEMICAL CO., LTD.) 15.08.2019 (2019-08-15), particularly, claims 1-6, examples 1-6 | 1-13 |
| P, A | WO 2019/102992 A1 (SEKISUI CHEMICAL CO., LTD.) 31.05.2019 (2019-05-31), particularly, claims 1-6, examples 3, 7-10 | 1-13 |

☐ Further documents are listed in the continuation of Box C.   ☒ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>08.06.2020 | Date of mailing of the international search report<br>23.06.2020 |
|---|---|
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

| INTERNATIONAL SEARCH REPORT | International application No. |
| Information on patent family members | PCT/JP2020/013934 |

| | |
|---|---|
| WO 2018/147333 A1  16.08.2018 | JP 2019-52135 A<br>US 2019/0117777 A1<br>particularly, claims 1-13,<br>examples 1-35,<br>paragraphs [0078], [0079]<br>JP 2019-52138 A<br>JP 6370520 B1<br>EP 3453404 A1<br>CN 109310777 A<br>AU 2018218868 A1<br>SG 11201906165P A<br>CA 3052757 A<br>KR 10-2019-0112266 A<br>MX 2019008972 A<br>TW 201831167 A |
| WO 2020/013241 A1  16.01.2020 | (Family: none) |
| WO 2019/155992 A1  15.08.2019 | (Family: none) |
| WO 2019/102992 A1  31.05.2019 | (Family: none) |

Form PCT/ISA/210 (patent family annex) (January 2015)

**REFERENCES CITED IN THE DESCRIPTION**

**Patent documents cited in the description**

- WO 2018147333 A